(19) Europäisches Patentamt
European Patent Office
Office européen des brevets

(11) Publication number: 0 687 668 A2

# (12) EUROPEAN PATENT APPLICATION

(21) Application number: 95303416.2

(51) Int. Cl.[6]: C07C 211/54, G03G 5/06

(22) Date of filing: 19.05.95

(30) Priority: 30.05.94 JP 117142/94

(43) Date of publication of application:
20.12.95 Bulletin 95/51

(84) Designated Contracting States:
DE FR GB IT

(71) Applicant: MITA INDUSTRIAL CO. LTD.
2-28, Tamatsukuri
1-chome, Chuo-ku, Osaka-shi
Osaka 540 (JP)

(72) Inventor: Miyamoto, Eiichi, c/o Mita Industrial Co., Ltd.
2-28, Tamatsukuri 1-chome,
Chuo-kuo
Osaka-shi, Osaka 540 (JP)
Inventor: Muto, Nariaki, c/o Mita Industrial Co., Ltd.
2-28, Tamatsukuri 1-chome,
Chuo-kuo
Osaka-shi, Osaka 540 (JP)
Inventor: Kakui, Mikio, c/o Mita Industrial Co., Ltd.
2-28, Tamatsukuri 1-chome,
Chuo-kuo
Osaka-shi, Osaka 540 (JP)
Inventor: Sumida, Keisuke, c/o Mita Industrial Co., Ltd.
2-28, Tamatsukuri 1-chome,
Chuo-kuo
Osaka-shi, Osaka 540 (JP)
Inventor: Fukami, Toshiyuki, c/o Mita Industrial Co., Ltd.
2-28, Tamatsukuri 1-chome,
Chuo-kuo
Osaka-shi, Osaka 540 (JP)
Inventor: Yamazato, Ichiro, c/o Mita Industrial Co., Ltd.
2-28, Tamatsukuri 1-chome,
Chuo-kuo
Osaka-shi, Osaka 540 (JP)
Inventor: Uegaito, Hisakazu, c/o Mita Industrial Co., Ltd.
2-28, Tamatsukuri 1-chome,
Chuo-kuo
Osaka-shi, Osaka 540 (JP)
Inventor: Tanaka, Yuji, c/o Mita Industrial Co., Ltd.
2-28, Tamatsukuri 1-chome,
Chuo-kuo
Osaka-shi, Osaka 540 (JP)

(74) Representative: W.P. THOMPSON & CO.
Eastcheap House
Central Approach
Letchworth, Hertfordshire SG6 3DS (GB)

## (54) m-Phenylenediamine derivatives and electrophotosensitive material using the same

(57) The present invention provides a m-phenylenediamine derivative which is novel as an electric charge transferring material, particularly hole transferring material, and an electrophosensitive material having a high sensitivity. This derivative includes a compound represented by the general formula (1) below or by formula (2) or (3) of the specification. Regarding the electrophotosensitive material, a photosensitive layer containing the m-phenylenediamine derivative as the hole transferring material is provided on a conductive substrate :

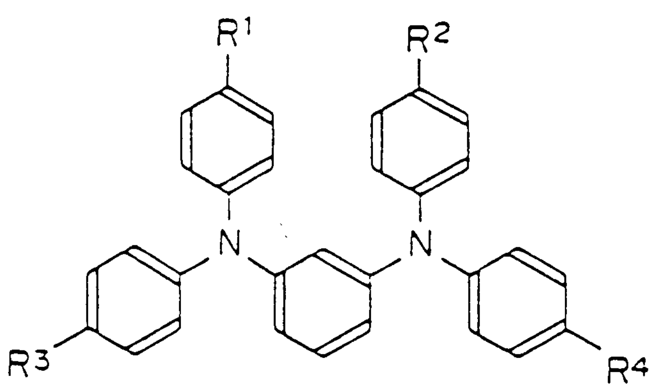

(1)



Jouve, 18, rue Saint-Denis, 75001 PARIS

wherein $R^1$ and $R^2$ are the same or different and each indicates a hydrogen atom or an alkyl group which may have one or more substituents ; and $R^3$ and $R^4$ are same or different and each indicates a straight-chain or branched alkyl group having 3 to 5 carbon atoms ; provided that $R^1$, $R^2$, $R^3$ and $R^4$ are not the same alkyl groups, simultaneously.

F I G. 1

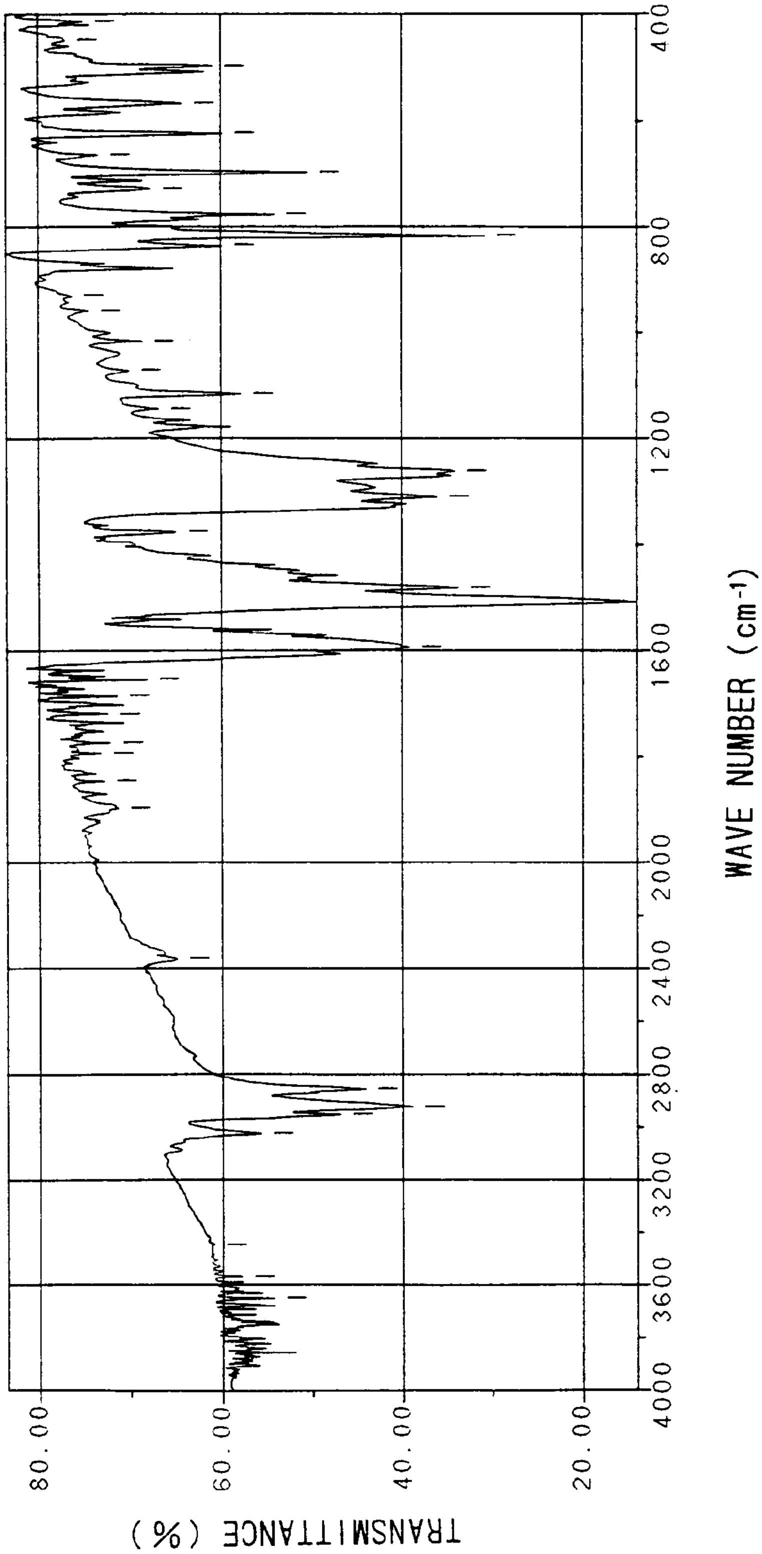

BACKGROUND OF THE INVENTION

The present invention relates to novel m-phenylenediamine derivatives which are suitably used as electric charge transferring materials (particularly, hole transferring materials) in applications such as solar batteries, electroluminescent devices, electrophotosensitive materials and the like, and an electrophotosensitive material using the same.

As the electric charge transferring material used for the above applications, there have been known various organic compounds such as carbazole compounds, oxadiazole compounds, pyrazoline compounds, hydrazone compounds, stilbene compounds, phenylenediamine compounds, benzidine compounds and the like.

These electric charge transferring materials are normally used in a state at which they are dispersed in a membrane of a suitable binding resin. In the case of electrophotosensitive material, for example, a so-called organic photoconductor (OPC) such as a single-layer type electrophotosensitive material comprising a single-layer type electrophotosensitive layer wherein the above electric charge transferring material and an electric charge generating material which generates an electric charge due to light irradiation are dispersed in a binding resin, a multi-layer type electrophotosensitive material comprising an electric charge transferring layer containing the above electric transferring material and an electric charge generating layer containing an electric charge generating layer, etc. are normally used. Such an organic photosensitive material has an advantage that it is superior in processability and is easily produced, thereby offering a great degree of freedom for design of performance.

Among these compounds, a m-phenylenediamine derivative represented by the general formula (4):

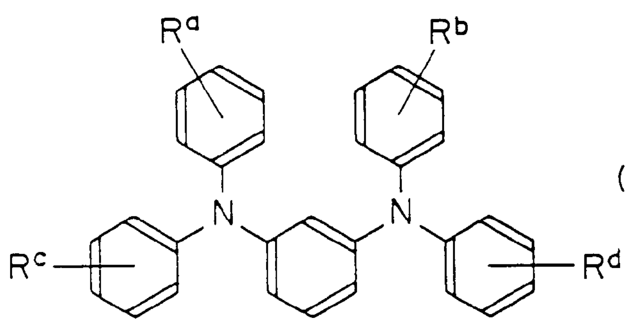

(4)

[wherein $R^a$, $R^b$, $R^c$ and $R^d$ are same or different and indicate a hydrogen atom, a methyl group or an ethyl group], which belongs to the phenylenediamine compound, has widely been used as the electric charge transferring material, particularly hole transferring material (see Japanese Laid-Open Patent Publication Nos. 142642/1989 and 261958/1991).

However, among conventional m-phenylenediamine derivatives described above, the compound represented by the formula (4a):

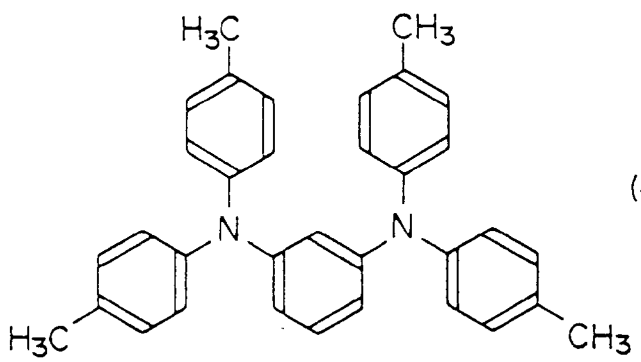

(4a)

wherein all substituents $R^a$ to $R^d$ in the formula (4) are methyl groups and all the phenyl groups are substituted at their 4-positions is inferior in compatibility with binding resin to cause a problem of crystallization, etc.

Further, the compound represented by the formula (4b):

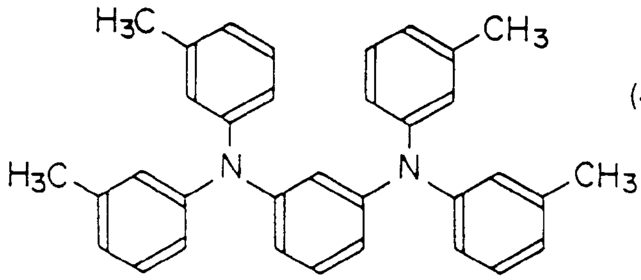

(4b)

wherein all substituents $R^a$ to $R^d$ are methyl groups and all the phenyl groups are substituted at their 3-positions, and the compound represented by the formula (4c):

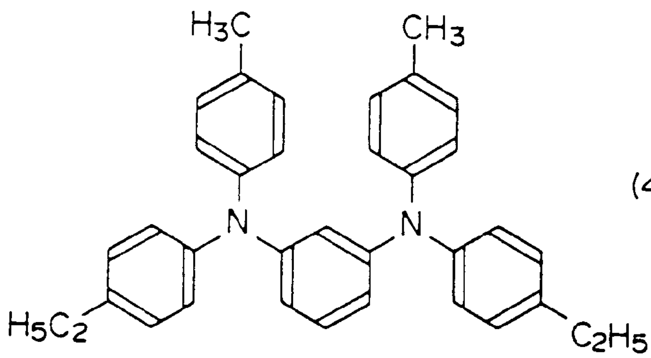

(4c)

wherein the substituents $R^a$ and $R^b$ are methyl groups which are substituted at 4-positions of their phenyl groups and the substituents $R^c$ and $R^e$ are ethyl groups which are substituted at the 4-positions of their phenyl groups do not cause crystallization as the above compound does, but are inferior in compatibility with binding resin as ever. Therefore, electrophotosensitive materials using these compounds have a problem that a sufficient hole transferring capability cannot be obtained and that it is difficult to realize a high sensitivity even if other high-performances materials, which constitute the photosensitive layer, are used.

## SUMMARY OF THE INVENTION

It is a main object of the present invention to provide a novel m-phenylenediamine derivative which can contribute to realisation of a higher sensitivity of the electrophotosensitive material.

It is another object of the present invention to provide an electrophotosensitive material having excellent sensitivity characteristics.

The m-phenylenediamine derivative of the present invention is represented by the general formula (1):

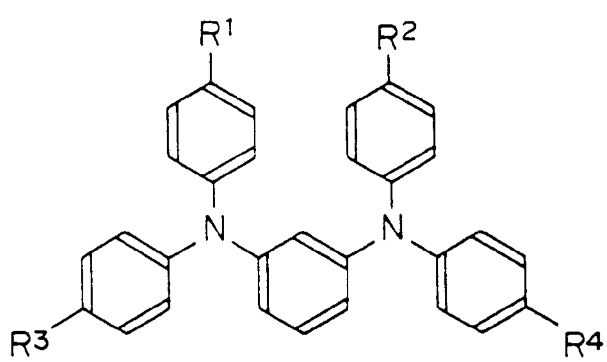

(1)

wherein $R^1$ and $R^2$ are the same or different and each indicates a hydrogen atom or an alkyl group which may contain one or more substituents; and $R^3$ and $R^4$ are same or different and each indicates a straight-chain or branched alkyl group having 3 to 5 carbon atoms; provided that $R^1$, $R^2$, $R^3$ and $R^4$ are not the same alkyl groups,

simultaneously; or the general formula (2):

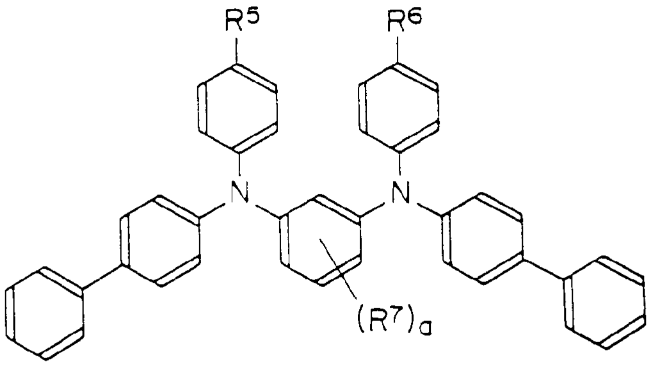

(2)

wherein $R^5$ and $R^6$ are the same or different and each indicates an alkyl group which may contain one or more substituents, an alkoxy group which may contain one or more substituents or a halogen atom; $R^7$ is a hydrogen atom, an alkyl group which may contain one or more substituents, an alkoxy group which may contain one or more substituents, an aryl group which may contain one or more substituents, a halogen atom, a cyano group or a nitro group; and a is an integer from 1 to 4.

Further, the electrophotosensitive material of the present invention comprises an electrophotosensitive layer containing a m-phenylenediamine derivative represented by the general formula (1) or (2), on a conductive substrate.

Another electrophotosensitive material of the present invention comprises a single-layer type photosensitive layer containing an electric charge generating material and a m-phenylenediamine derivative represented by the general formula (3):

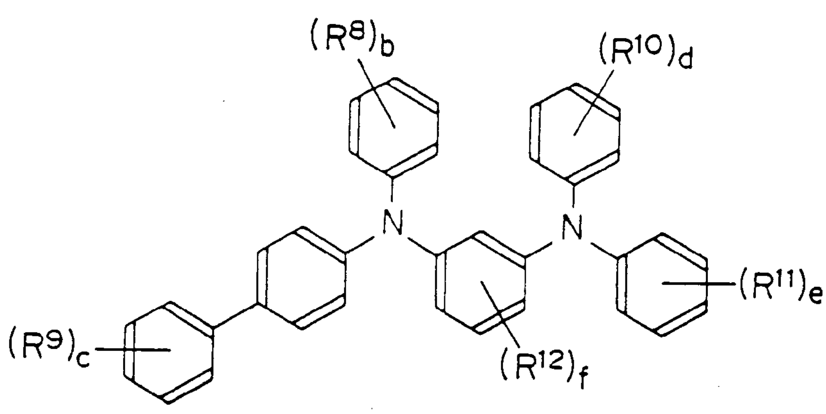

(3)

wherein $R^8$, $R^9$, $R^{10}$ and $R^{11}$ are the same or different and each indicates a hydrogen atom, an alkyl group which may contain one or more substituents, an alkoxy group which may contain one or more substituents or a halogen atom; $R^{12}$ is a hydrogen atom, an alkyl group which may contain one or more substituents, an alkoxy group which may contain one or more substituents, an aryl group which may contain one or more substituents, a halogen atom, a cyano group or a nitro group; b, c, d and e independently indicate an integer from 1 to 5; and f is an integer from 1 to 4, as an electric charge transferring material, on a conductive substrate.

Regarding the m-phenylenediamine derivative represented by the general formula (1) of the present invention, its compatibility with binding resin is further improved in comparison with a conventional m-phenylenediamine derivative, by substituting at the 4-positions of two phenyl groups among four phenyl groups at the exterior of a m-phenylenediamine skeleton with straight-chain or branched alkyl groups having 3 to 5 carbon atoms, respectively. Also, the m-phenylenediamine derivative (1) exhibits good matching with other materials constituting the photosensitive layer, particularly electric charge generating material, to pour holes generated in the electric charge transferring material, smoothly, and forms no ion trap which can be the cause of deterioration in sensitivity.

Accordingly, regarding the electrophotosensitive material containing the m-phenylenediamine derivative (1) of the present invention, the hole transferring capability of the photosensitive layer is improved and, therefore, it becomes possible to realize high sensitivity.

When the substituents $R^1$, $R^2$, $R^3$ and $R^4$ are the same alkyl groups in the general formula (1), the symmetry of the molecule is too high and, therefore, the compatibility with binding resin is lowered and the hole transferring capability of the photosensitive layer cannot be improved. Therefore, the above case should be excluded from the present invention. Incidentally, for example, the case wherein three substituents among $R^1$, $R^2$, $R^3$ and $R^4$ are n-propyl groups and the other substituent is an isopropyl group is included in the scope of the present invention.

On the other hand, regarding the m-phenylenediamine derivative represented by the general formula (2) of the present invention, the mobility of holes is improved by substituting two phenyl groups among four phenyl groups at the exterior of the m-phenylenediamine skeleton with 4-biphenylyl groups and substituting 4-positions of other two phenyl groups with any substituents and, at the same time, the compatibility with binding resin is further improved by substituting 4-biphenylyl groups with no substituent. Further, since the m-phenylenediamine derivative (2) has a large molecular weight in comparison with a conventional derivative, it is superior in stability. Also, the m-phenylenediamine derivative (2) exhibits good matching with other materials constituting the photosensitive layer, particularly electric charge generating material, to pour holes generated in the electric charge transferring material, smoothly, and forms no ion trap which can be the cause of deterioration in sensitivity, similar to the m-phenylenediamine derivative (1).

Accordingly, regarding the electrophotosensitive material containing the m-phenylenediamine derivative (2) of the present invention, the hole transferring capability of the photosensitive layer is improved and, therefore, it becomes possible to realize high sensitivity. Also, it is superior in stability.

Furthermore, regarding the m-phenylenediamine derivative represented by the general formula (3) of the present invention, the mobility of holes is improved by substituting at least one phenyl group among four phenyl groups at the exterior of the m-phenylenediamine skeleton with a 4-biphenylyl group. Further, since the m-phenylenediamine derivative (3) has a large molecular weight in comparison with a conventional derivative, it is superior in stability. Also, the m-phenylenediamine derivative (3) exhibits good matching with other materials (particularly charge generating material) constituting the photosensitive layer, (particularly single-layer type photosensitive layer) to pour holes generated in the charge generating material, smoothly, and forms no ion trap which can be the cause of deterioration in sensitivity, similar to the m-phenylenediamine derivatives (1) and (2).

Accordingly, regarding the electrophotosensitive material comprising the single-layer type photosensitive layer containing the m-phenylenediamine derivative (3) of the present invention, the hole transferring capability of the single-layer type photosensitive layer is improved and, therefore, it becomes possible to realize high sensitivity. Also, it is superior in stability.

## BRIEF DESCRIPTION OF THE DRAWINGS

Fig. **1** is a graph illustrating the results of infrared spectroscopic analysis of the m-phenylenediamine derivative obtained in Synthesis Example 1.

Fig. **2** is a graph illustrating the results of infrared spectroscopic analysis of the m-phenylenediamine derivative obtained in Synthesis Example 2.

Fig. **3** is a graph illustrating the results of infrared spectroscopic analysis of the m-phenylenediamine derivative obtained in Synthesis Example 3.

Fig. **4** is a graph illustrating the results of infrared spectroscopic analysis of the m-phenylenediamine derivative obtained in Reference Example 1.

## DETAILED DESCRIPTION OF THE INVENTION

In the m-phenylenediamine derivative represented by the general formula (1) of the present invention, the substituents $R^3$ and $R^4$ in the formula are specifically limited to straight-chain or branched alkyl groups having 3 to 5 carbon atoms, as described above. When the number of carbon atoms of the alkyl group is less than 3, good compatibility with binding resin cannot be obtained. On the other hand, when the number of the carbon atoms of the alkyl group exceeds 5, the hole transferring capability is hindered.

Examples of the alkyl group corresponding to the groups $R^3$ and $R^4$ in the general formula (1) include a normal propyl group (n-Pr), an isopropyl group (i-Pr), a normal butyl group (n-Bu), an isobutyl group (i-Bu), a tert-butyl group (t-Bu), a normal pentyl group (n-Pe) and the like.

Further, examples of the alkyl group corresponding to the groups $R^1$ and $R^2$ include lower alkyl groups having 1 to 6 carbon atoms, such as a methyl group, an ethyl group, a normal propyl group, an isopropyl group, a butyl group, an isobutyl group, a tert-butyl group, a pentyl group, a hexyl group and the like. Among them, methyl and ethyl groups are suitably used.

Incidentally, as described above, when the groups $R^3$ and $R^4$ are the same alkyl group, at least one of the groups $R^1$ and $R^2$ must be another alkyl group. Preferably both $R^1$ and $R^2$ are another alkyl group; more preferably $R^1$ and $R^2$ are the same alkyl group. For example, when the groups $R^3$ and $R^4$ are normal propyl groups, simultaneously, at least one of the groups $R^1$ and $R^2$ must be a hydrogen atom or an alkyl group (including an isopropyl group and a normal propyl group containing a substituent) other than a normal propyl group. Preferably both $R^1$ and $R^2$ are hydrogen or a said alkyl group. Further, when the groups $R^3$ and $R^4$ are isopropyl groups, simultaneously, at least one of the groups $R^1$ and $R^2$ must be a hydrogen atom or an alkyl group (including a normal propyl group and an isopropyl group containing a substituent group) other than an isopropyl group. Preferably both $R^3$ and $R^4$ are hydrogen or a said alkyl group.

Examples of the substituent with which the alkyl group corresponding to the groups $R^1$ and $R^2$ may be substituted include a halogen atom, an amino group, a cyano group, a nitro group and the like. Any number of substituents can be substituted on any position of the alkyl group.

Examples of the m-phenylenediamine derivative of the present invention include compounds represented by the following formulas (1a) to (1c).

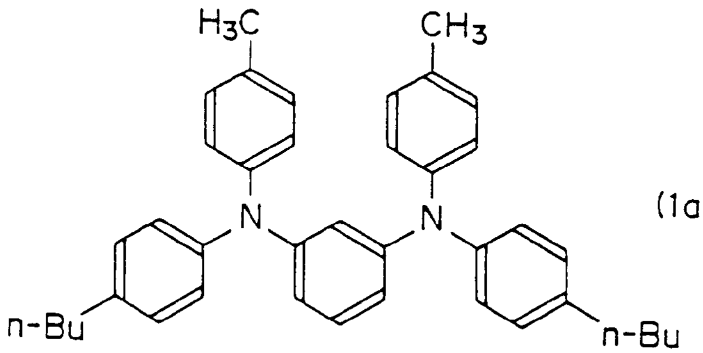

(1a)

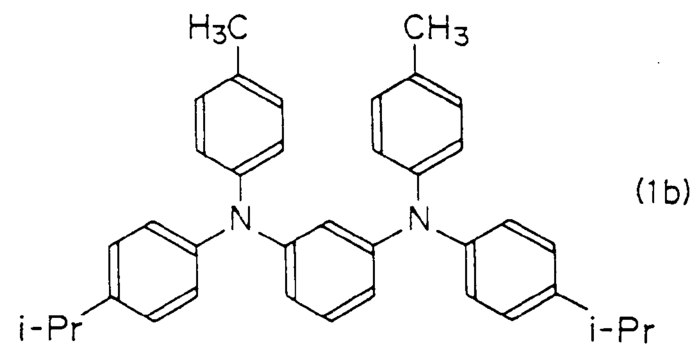

(1b)

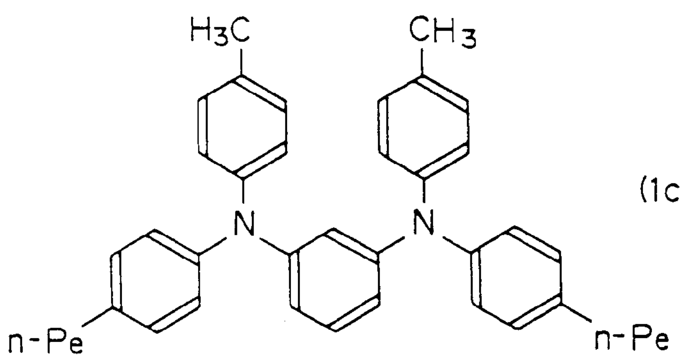

(1c)

The m-phenylenediamine derivative of the present invention can be synthesized by various methods. For example, the m-phenylenediamine derivative of the formula (1a) can be synthesized according to the following reaction scheme.

That is, N,N'-diacetyl-1,3-phenylenediamine represented by the following formula (a) is firstly mixed with

4-methyliodobenzene represented by the following formula (b) in a molar ratio of 1:2, together with copper powder, copper oxide or halogenated copper, and the mixture is reacted in the presence of a basic substance to give N,N'-diacetyl-N,N'-bis(4-tolyl)-1,3-phenylenediamine represented by the following formula (c).

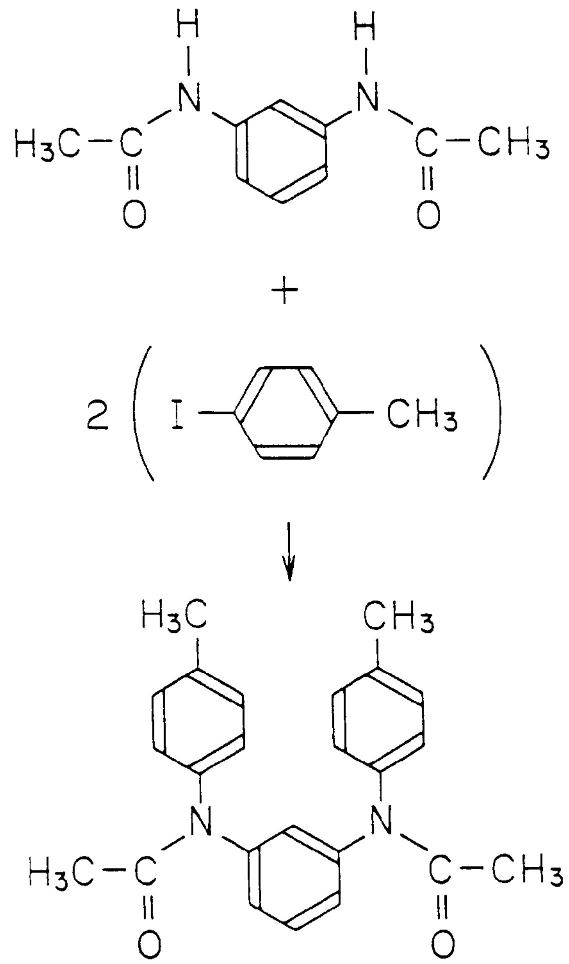

(a)

(b)

(c)

Then, N,N'-diacetyl-N,N'-bis(4-tolyl)-1,3-phenylenediamine of the formula (c) is subjected to the deacetylation reaction to give N,N'-bis(4-tolyl)-1,3-phenylenediamine represented by the following formula (d), which is reacted with 4-normal butyliodobenzene in a molar ratio of 1:2 according to the same manner as that described above to synthesize a m-phenylenediamine derivative of the formula (1a).

The m-phenylenediamine derivative other than that of the formula (1a) can also be synthesized under the same condition according to the following reaction scheme.

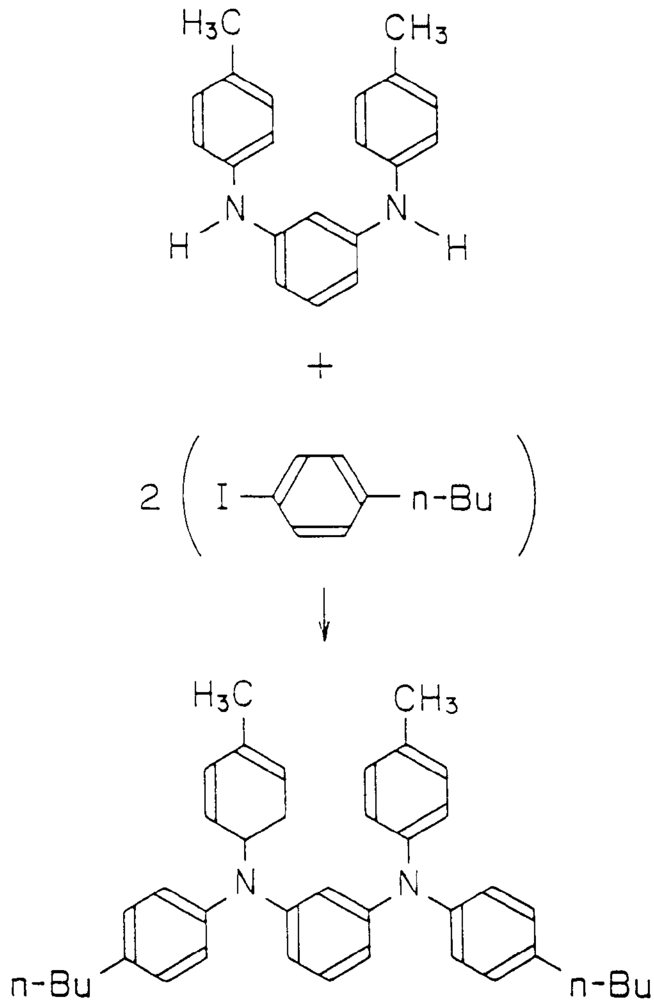

(d)

(e)

(1a)

As described above, the above m-phenylenediamine derivative of the present invention can be suitably used as an electric charge transferring material, particularly hole transferring material, in applications such as solar batteries electroluminescent devices, electrophotosensitive materials and the like, and it can also be used in other various fields.

Next, in another m-phenylenediamine derivative represented by the general formula (2) of the present invention, examples of the alkyl group corresponding to the groups $R^5$, $R^6$ and $R^7$ in the formula include the same lower alkyl groups having 1 to 6 carbon atoms as those described as to the groups $R^1$ and $R^2$ in the general formula (1).

Examples of the alkoxy group corresponding to the groups $R^5$, $R^6$ and $R^7$ include alkoxy groups having 1 to 6 carbon atoms, such as a methoxy group, an ethoxy group, a propoxy group, a t-butoxy group, a pentyloxy group, a hexyloxy group and the like. Examples of the halogen atom include a chlorine atom, a bromine atom, a fluorine atom, an iodine atom and the like.

Examples of the aryl group corresponding to the group $R^7$ include a phenyl group, a tolyl group, a xylyl group, a biphenylyl group, a naphthyl group, an anthryl group,a phenanthryl group and the like.

Examples of the substituent with which the alkyl group, alkoxy group and aryl group may be substituted include a halogen atom, an amino group, a cyano group, a nitro group and the like. Any number of substituentscan be substituted on any position of the alkyl group.

Examples of the m-phenylenediamine derivative of the present invention include compounds represented by the following formulas (2a) to (2i).

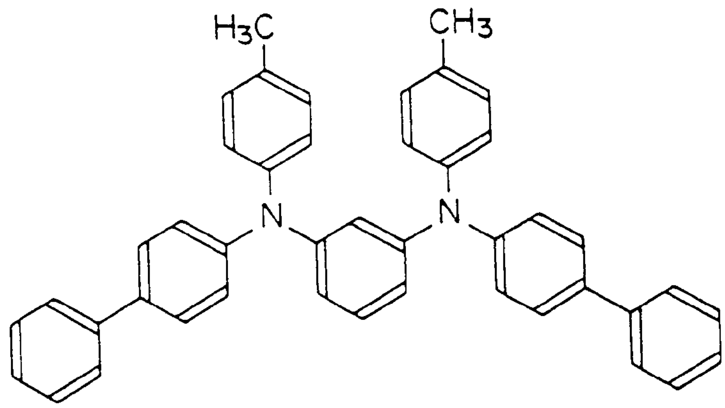

(2a)

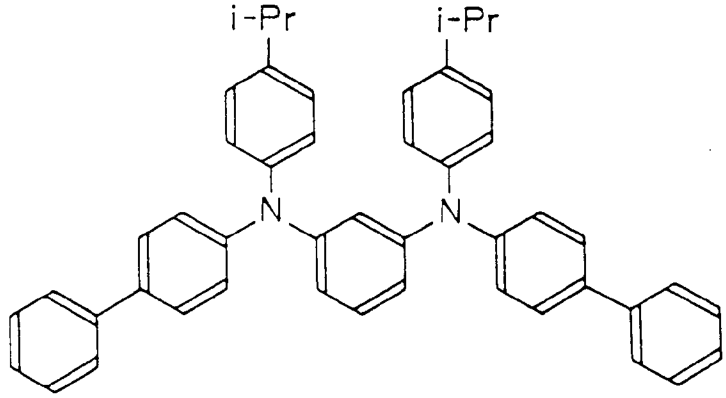

(2b)

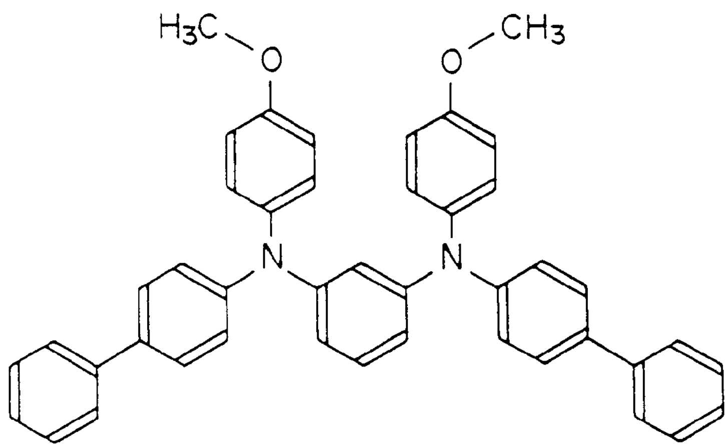

(2c)

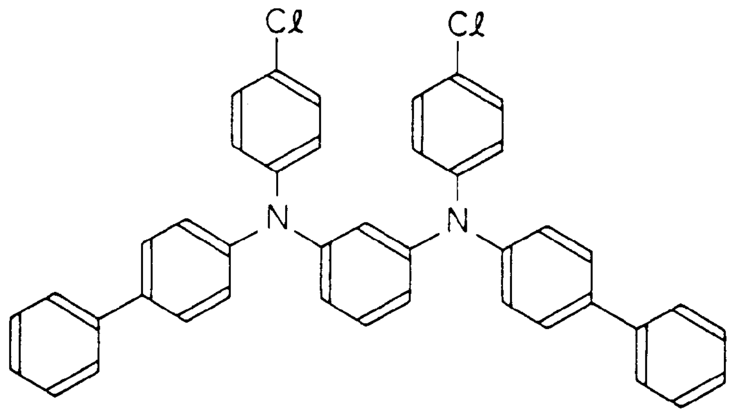

(2d)

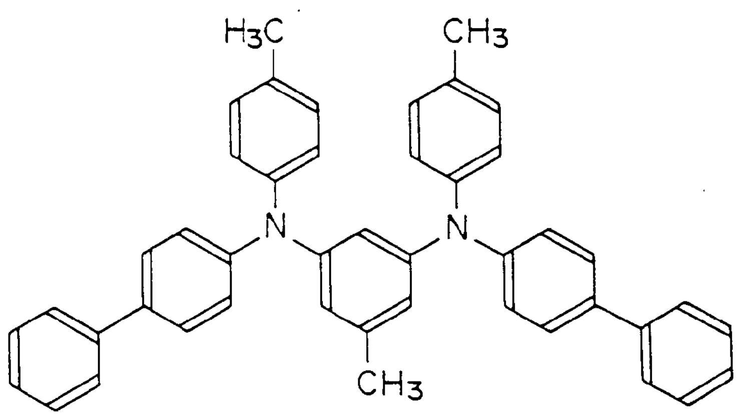

(2e)

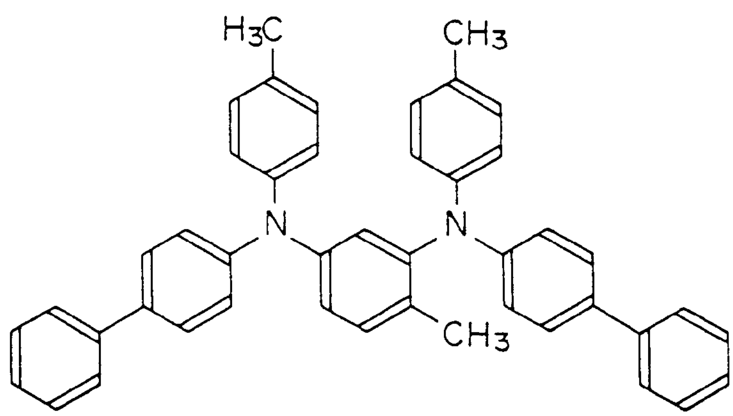

(2f)

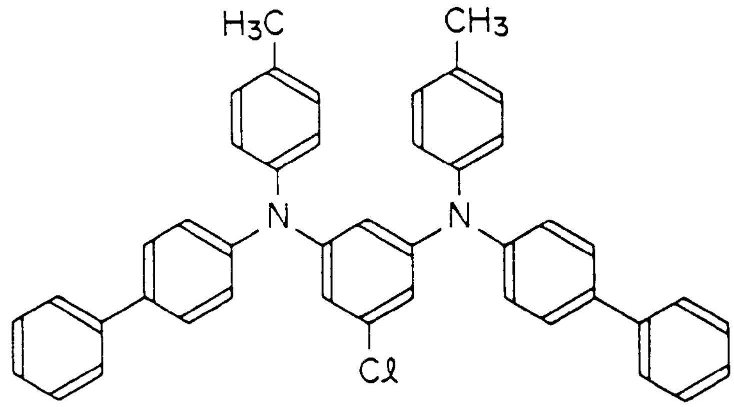

(2g)

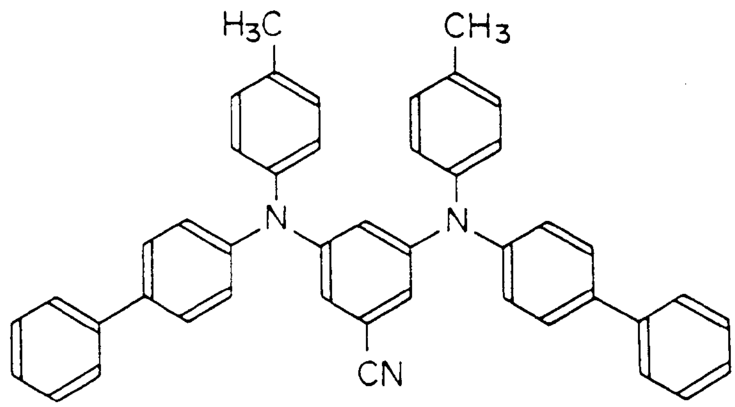

(2h)

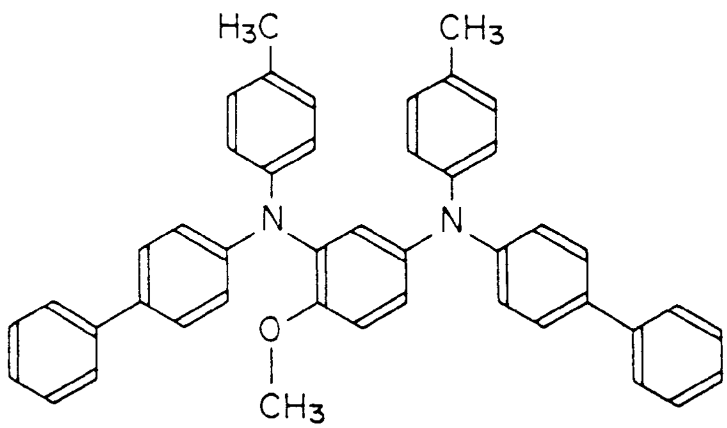

(2i)

The m-phenylenediamine derivative (2) of the present invention can be synthesized by various methods, particularly, it can be synthesized according to the same reaction scheme as that of the m-phenylenediamine derivative of the general formula (1).

For example, the m-phenylenediamine derivative of the formula (2a) is synthesized by reacting N,N'-bis(4-tolyl)-1,3-phenylenediamine represented by the formula (d), which is obtained according to the above reaction scheme, with 4-phenyliodobenzene represented by the following formula (f) in a molar ratio of 1:2 according to the same manner as that described above.

The m-phenylenediamine derivative other than that of the formula (2a) can also be synthesized under the same condition according to the following reaction scheme.

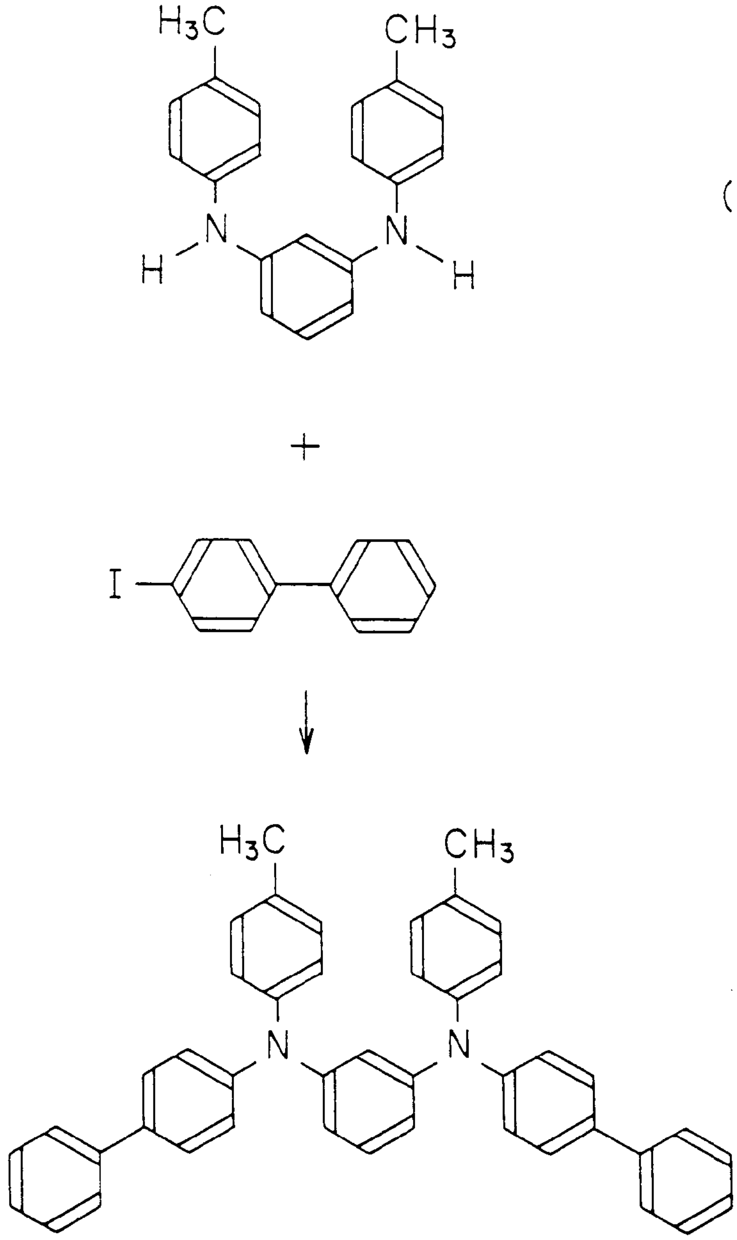

(d)

(f)

(2a)

The above m-phenylenediamine derivative of the present invention can also be suitably used as an electric charge transferring material, particularly hole transferring material, in applications such as solar batteries, electroluminescent devices, electrophotosensitive materials and the like, and it can also be used in other various fields.

Furthermore, in the m-phenylenediamine derivative represented by the general formula (3), examples of the alkyl group, alkoxy group, aryl group and halogen atom corresponding to the groups $R^8$, $R^9$, $R^{19}$, $R^{11}$ and $R^{12}$ and examples of the substituent with which the alkyl group, alkoxy group and aryl group may be substituted include the same groups as those described above.

Examples of the m-phenylenediamine derivative represented by the general formula (3) include compounds represented by the following formulas (3a) to (3g).

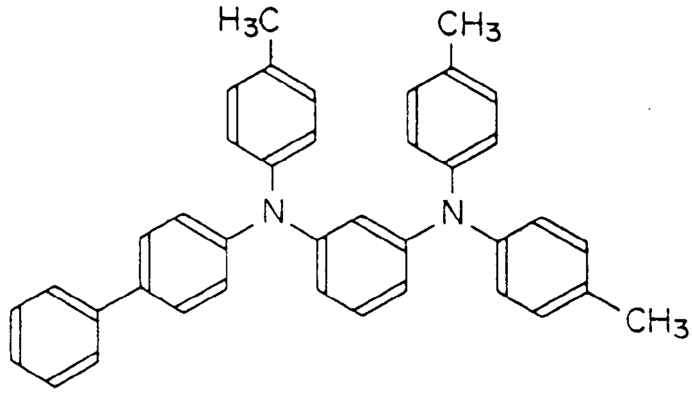

(3a)

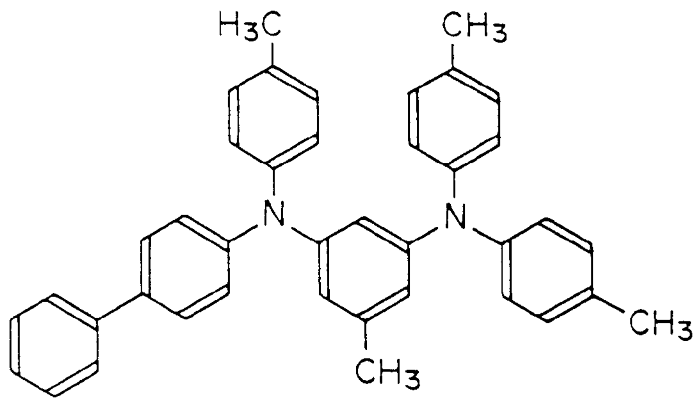

(3b)

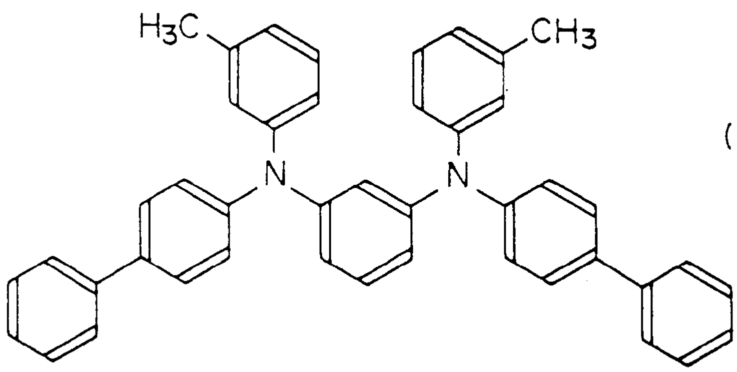

(3c)

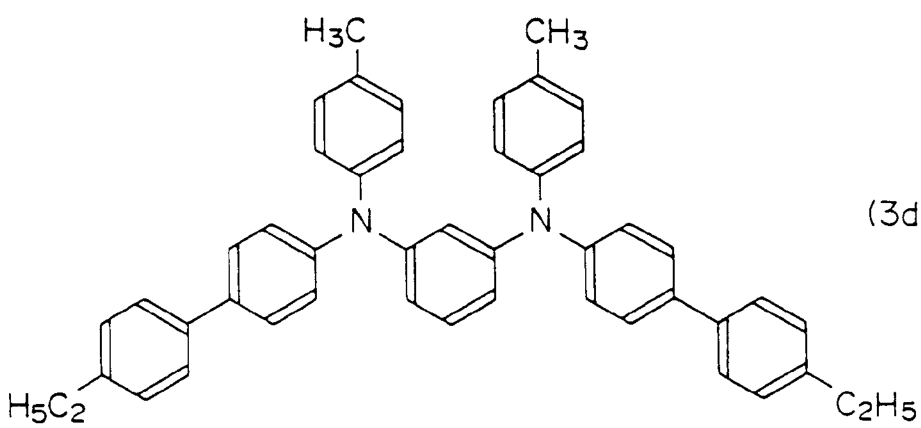

(3d)

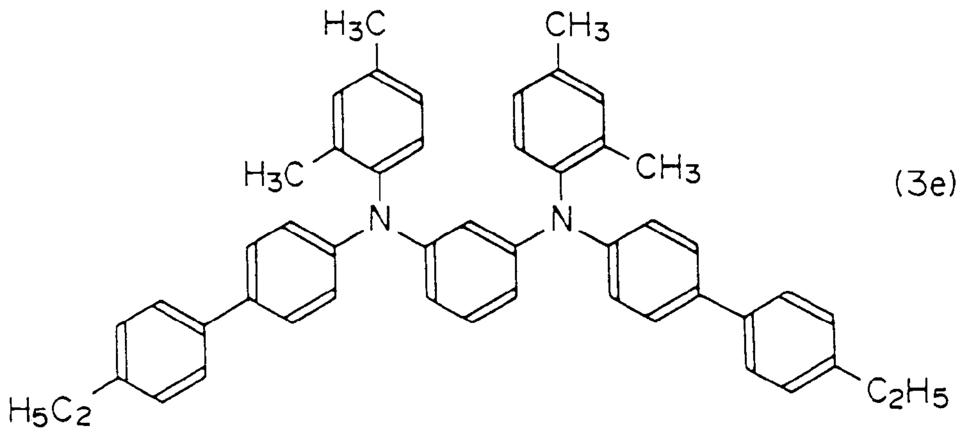

(3e)

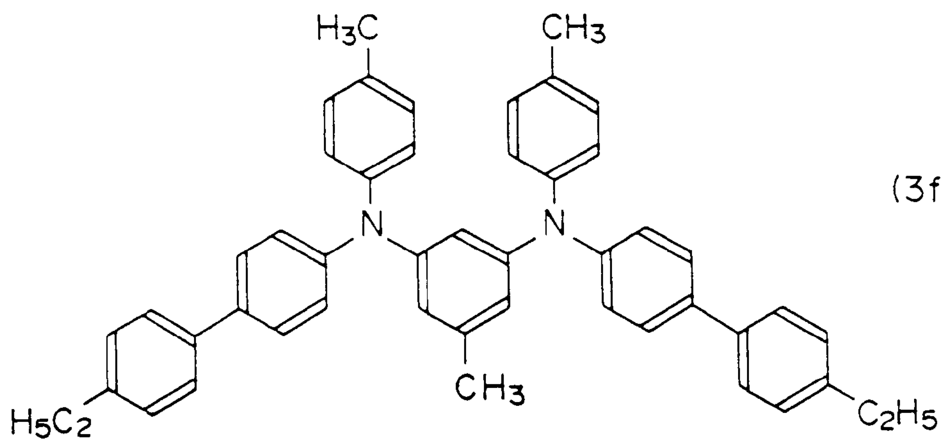

(3f)

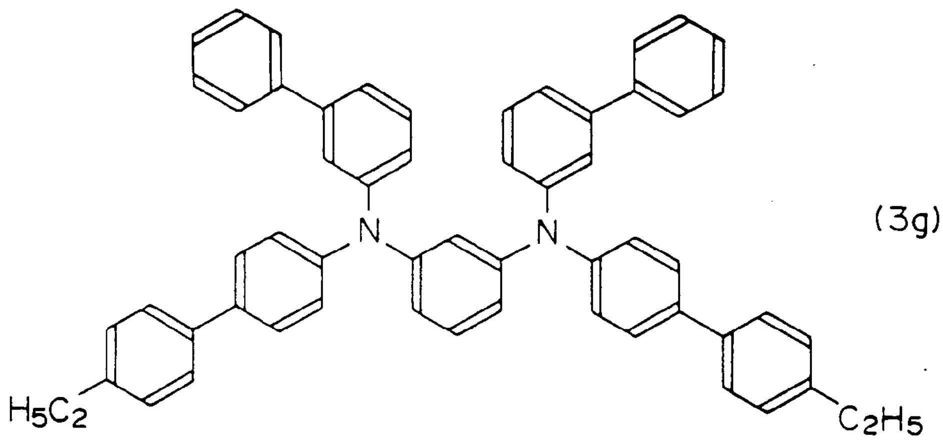

(3g)

The electrophotosensitive material of the present invention comprises an electrophotosensitive layer containing one or more sorts of phenylenediamine derivatives represented by any one of the formulas (1) to (3), on a conductive substrate. The electrophotosensitive layer is classified into two types, i.e. a single-layer type electrophotosensitive layer and a multi-layer type electrophotosensitive layer. When using the m-phenylenediamine derivative represented by the formula (1) or (2) of the present invention, any layer construction may be adopted. Further, when using the m-phenylenediamine derivative represented by the formula (3), the effect of the m-phenylenediamine derivative is remarkably exhibited in the single-layer type photosensitive layer, as described above, so that the photosensitive layer is limited to the single-layer type.

The single-layer type photosensitive layer can be formed by applying a coating solution, which is prepared by dissolving or dispersing a m-phenylenediamine derivative represented by any one of the general formulas (1) to (3) as a hole transferring material, an electric charge generating material and a binding resin in a suitable

solvent, on a conductive substrate using a means such as application, followed by drying.

Further, the multi-layer type photosensitive layer may be formed by forming an electric charge generating layer containing an electric charge generating material on a conductive substrate using a means such as deposition or application, applying a coating solution containing a m-pheneylenediamine derivative represented by the general formula (1) or (2) as a hole transferring material and a binding resin on the electric charge generating layer using a means such as application, followed by drying to form an electric charge transferring layer. To the contrary, an electric charge transferring layer may be formed on a conductive substrate and then an electric charge generating layer may be formed thereon.

Examples of the electric charge generating material include powder of inorganic conductive materials (e.g. selenium, selenium-tellurium, selenium-arsenic, cadmium sulfide, α-silicon, etc), azo pigments, perylene pigments, anthanthrone pigments, phthalocyanine pigments, indigo pigments, triphenylmethane pigments, therene pigments, toluidine pigments, pyrazoline pigments, quinacridon pigments, dithioketopyrrolopyrrole pigments and the like. Among them, phthalocyanine pigments are preferred for an electrophotosensitive material which is suitable for a digital optical system image forming apparatus using a light source having a wavelength of 700 nm or more, and perylene pigments and azo pigments are preferred for an electrophotosensitive material which is suitable for an analog optical system image forming apparatus using a light source within the visible range. These electric charge generating materials can be used alone or in combination thereof according to the range of sensitivity of the electrophotosensitive material.

Examples of the phthalocyanine pigment include compounds represented by the following formulas (G1) and (G2).

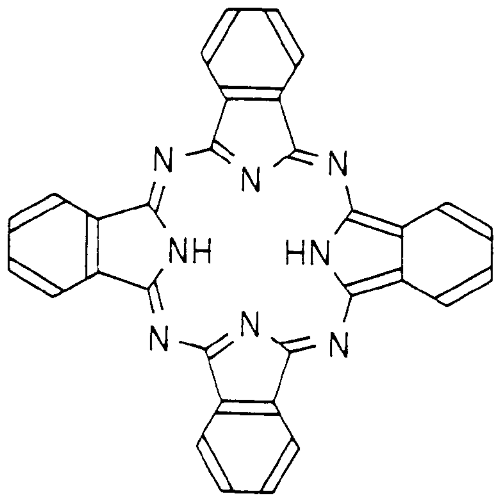

(G1)

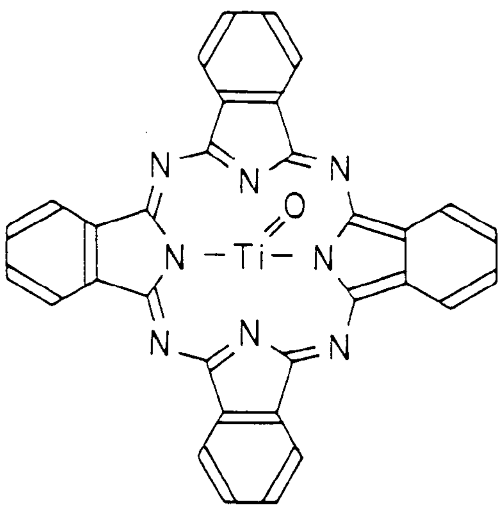

(G2)

Examples of the perylene pigment include a compound represented by the following formula (G3):

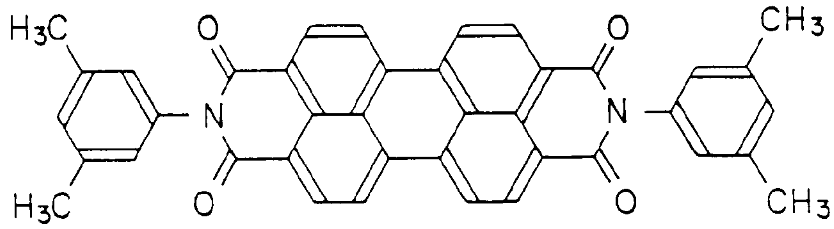

(G3)

Furthermore, examples of the azo pigment include compounds represented by the following formulas (G4) to (G10).

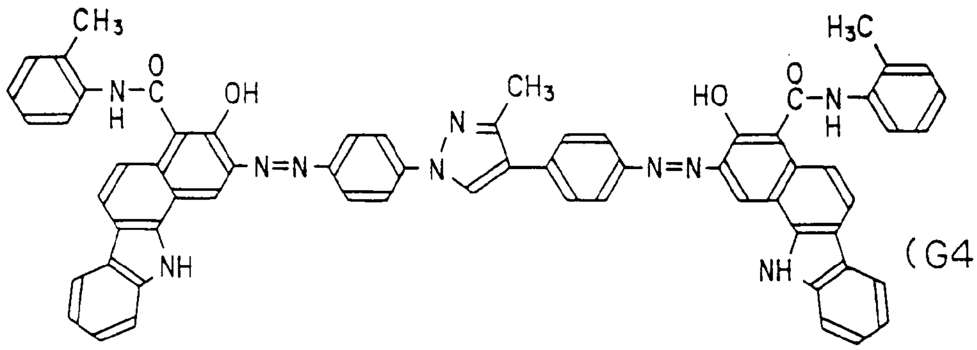

(G4)

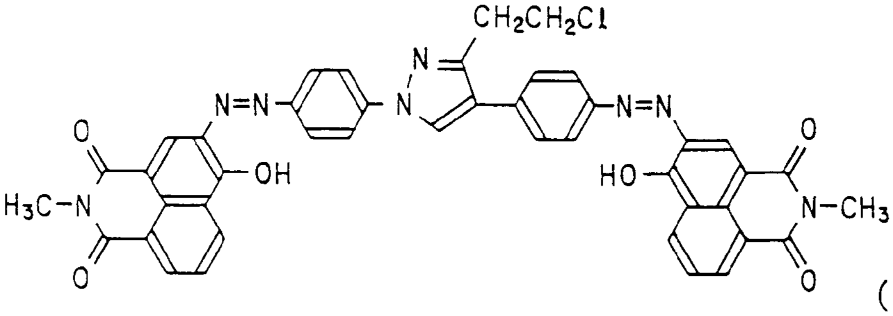

(G5)

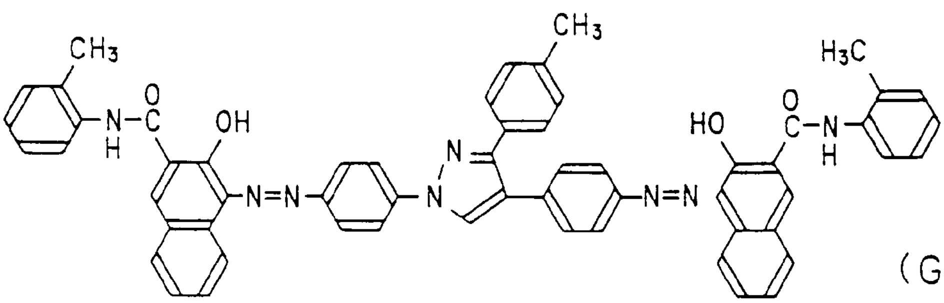

(G6)

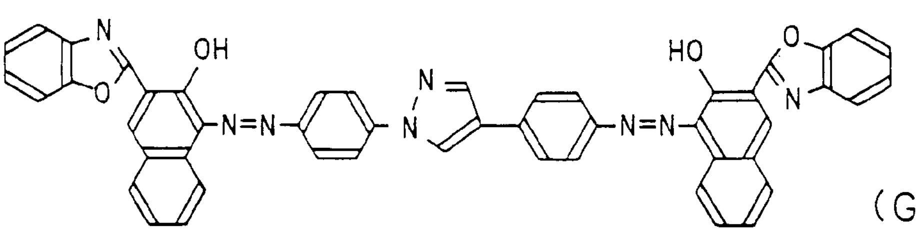

(G7)

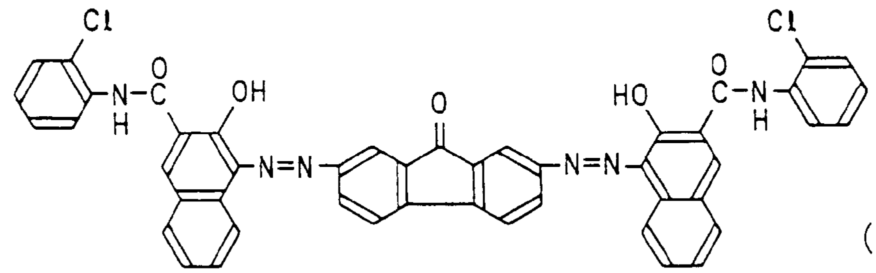

(G8)

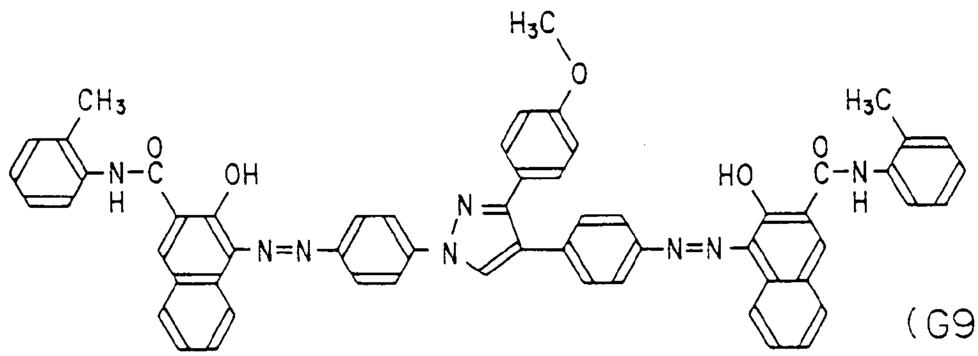

(G9)

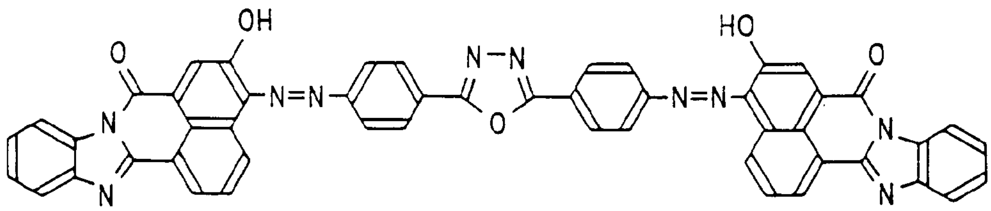

(G10)

The m-phenylenediamine derivative represented by any one of the general formulas (1) to (3) as the hole transferring material can be used alone or in combination with the other electric charge transferring material which has hitherto been known.

Examples of the other electric charge transferring material include various electron transferring materials and hole transferring materials.

Examples of the electron transferring material among electric charge transferring materials include electron attractive materials such as diphenoquinone compounds, benzoquinone compounds, naphthoquinone compounds, malononitrile, thiopyran compounds, tetracyanoethylene, tetracyanoquinodimethane, chloranil, bromanil, 2,4,7-trinitro-9-fluorenone, 2,4,5,7-tetranitro-9-fluorenone, 2,4,7-trinitro-9-dicyanomthylenefluorenone, 2,4,5,7-tetranitroxanthone, 2,4,8-trinitrothioxanthone, dinitrobenzene, dinitroanthracene, dinitroacridine, nitroanthraquinone, dinitroanthraquinone, succinic anhydride, maleic anhydride, dibromomaleic anhydride, etc., high-molecular electron attractive materials and the like.

Examples of the hole transferring material include electron donative materials such as diamine compounds other than the m-phenylenediamine derivatives represented by the general formulas (1) to (3); diazole compounds such as 2,5-bis(4-methylaminophenyl)-1,3,4-oxadiazole; styryl compounds such as 9-(4-diethylaminostyryl)anthracene, etc.; carbazole compounds such as polyvinyl carbazole; pyrazoline compounds such as 1-phenyl-3-(p-dimethylaminophenl)pyrazoline; hydrazone compounds; triphenylamine compounds; nitrogen-containing cyclic compounds and condensed polycyclic compounds which include indol compounds, oxazole compounds, isooxazole compounds, thiazole compounds, thiadiazole compounds, imidazole compounds, pyrazole compounds, triazole compounds and the like.

These electric charge transferring can also be used alone or in combination thereof. Further, when using an electric charge transferring material having film forming properties such as polyvinyl carbazole, the binding resin is not necessarily required.

Examples of the binding resin include thermoplastic resins such as styrene polymer, styrene-butadiene copolymer, styrene-acrylonitrile copolymer, styrene-maleic acid copolymer, acrylic polymer, styrene-acrylic copolymer, polyethylene, ethylene-vinyl acetate copolymer, chlorinated polyethylene, polyvinyl chloride, polypropylene, vinyl chloride-vinyl acetate copolymer, polyester, alkyd resin, polyamide, polyurethane, polycarbonate, polyalylate, polysulpfone, diallyl phthalate resin, ketone resin, polyvinyl butyral resin, polyether resin; crosslinking thermosetting resins such as silicone resin, epoxy resin, phenol resin, urea resin, melamine resin; photosetting resins such as epoxy-acrylate, urethane-acrylate, etc. These binding resins can be used alone or in combination thereof.

Additives such as sensitizers, fluorene compounds, antioxidants, ultraviolet absorbers, plasticizers, surfactants, leveling agents can be added to the photosensitive layer, in addition to the above respective components. In order to improve sensitivity of the photosensitive material, there may be used sensitizers such as terphenyl, halonaphthoquinones, acenaphthylene, in combination with the electric charge generating material.

In the multi-layer photosensitive material, the electric charge generating material constituting the electric charge generating layer and the binding resin may be used in various proportions. It is preferred that 5 to 1000 parts by weight, particularly 30 to 500 parts by weight of the electric charge generating material is used, based on 100 parts by weight of the binding resin.

The hole transferring material constituting the electric charge transferring layer and the binding resin can be used in various proportions within such a range as not to prevent the transmission of the electric charge and as to prevent the crystallization of the electric charge transferring material. It is preferred that 10 to 500 parts by weight, particularly 25 to 200 parts by weight of the electric charge transferring material containing the m-phenylenediamine derivative represented by any one of the general formulas (1) to (3) as the hole transferring material is used, based on 100 parts by weight of the binding resin so as to easily transfer the electric charge generated in the electric charge generating layer due to light irradiation.

Regarding the thickness of the multi-layer type electrophotosensitive layer, it is preferred that the thickness of the electric charge generating layer is about 0.01 to 5 μm, particularly about 0.1 to 3 μm and the thickness of the electric charge transferring layer is about 2 to 100 μm, particularly about 5 to 50 μm.

In the single-layer type electrophotosensitive material, it is preferred that 0.1 to 50 parts by weight, particularly 0.5 to 30 parts by weight of the electric charge generating material and 10 to 500 parts by weight, particularly 25 to 200 parts by weight of the hole transferring material containing the m-phenylenediamine derivative represented by any one of the general formulas (1) to (3) are used, based on 100 parts by weight of the binding resin. When the electron transferring material is used in combination, it is preferred that 5 to 100 parts by weight, particularly 10 to 80 parts by weight of the electron transferring material is used, based on 100 parts by weight of the binding resin. The total amount of the hole transferring material and electron transferring material is 20 to 500 parts by weight, particularly 30 to 200 parts by weight, based on 100 parts by weight of the binding resin.

It is preferred that the film thickness of the single-layer type photosensitive layer is 5 to 100 μm, particularly 10 to 50 μm.

A barrier layer may be formed, in such a range as not to injure the characteristics of the electrophotosensitive material, between the conductive substrate and the electrophotosensitive layer in the single-layer type electrophotosensitive material, or between the conductive substrate and the electric charge generating layer, between the conductive substrate and the electric charge transferring layer or between the electric charge generating layer and the electric charge transferring layer in the multi-layer type electrophotosensitive material. Further, a protective layer may be formed on the surface of the photosensitive material.

As the conductive substrate on which the above respective layers are formed, various materials having conductivity can be used, and examples thereof include metals such as aluminum, copper, tin, platinum, silver, vanadium, molybdenum, chromium, cadmium, titanium, nickel, palladium, indium, stainless steel, brass; plastic materials vapor-deposited or laminated with the above metal; glass materials coated with aluminum iodide, tin oxide, indium oxide, etc.

The conductive substrate may be made in the form of a sheet or a drum. The substrate itself may have a conductivity or only the surface of the substrate may have a conductivity. It is preferred that the conductive substrate has a sufficient mechanical strength when used.

When the above respective layer are formed by a coating method, the electric charge generating material, the electric charge transferring material, the binding resin, etc. may be dispersed and mixed with a suitable solvent by a known method, for example, using a roll mill, a ball mill, an atriter, a paint shaker, a supersonic dispenser, etc. to prepare a coating solution, which is applied by a known means and then allowed to dry.

As the solvent for preparing the coating solution, there can be used various organic solvents, and examples thereof include alcohols such as methanol, ethanol, isopropanol, butanol; aliphatic hydrocarbons such as n-hexane, octane, cyclohexane; aromatic hydrocarbons such as benzene, toluene, xylene; halogenated hydrocarbons such as dichloromethane, dichloroethane, carbon tetrachloride, chlorobenzene; ethers such as dimethyl ether, diethyl ether, tetrahydrofuran, ethylene glycol dimethyl ether, diethylene glycol dimethyl ether; ketones such as acetone, methyl ethyl ketone, cyclohexanone; esters such as ethyl acetate, methyl acetate; dimethylformaldehyde, dimethylformamide, dimethylsulfoxide, etc. These solvents may be used alone or in combinations thereof.

In order to improve a dispersibility of the electric charge transferring material and electric charge generating material as well as a smoothness of the surface of the photosensitive layer, there may be added surfactants, leveling agents, etc. to the coating solution.

As described above, the m-phenylenediamine derivative of the present invention has a high hole transferring capability and is also superior in compatibility with binding resin. Accordingly, the m-phenylenediamine derivative of the present invention can be suitably used as an electric charge transferring material, particularly hole transferring material, in applications such as solar batteries electroluminescent devices, electrophotosensitive materials and the like.

Further, the electrophotosensitive material of the present invention comprises a photosensitive layer containing the above m-phenylenediamine derivative as the hole transferring material and, therefore, it has a high sensitivity and is superior in durability and heat resistance.

EXAMPLES

The following Synthesis Examples, Examples and Comparative Examples further illustrate the present invention in detail.

M-phenylenediamine derivative (1)

Synthesis Example 1

9.6 g of N,N'-diacetyl-1,3-phenylenediamine represented by the formula (a), 21.8 g of 4-methyliodobenzene of the above formula (b), 13.8 g of potassium carbonate and 1 g of copper powder were added in 150 ml of nitrobenzene, and the mixture was refluxed while a nitrogen gas was blowing into this reaction system under vigorous stirring for 24 hours. The water produced in the reaction was removed out of the reaction system by azeotropic distillation with nitrobenzene.

After the reaction solution was cooled, the inorganic substance was filtered off. Further, nitrobenzene was distilled off by steam distillation to give the residue, which was added in 100 ml of tetrahydrofuran, together with 10 % hydrochloric acid. The mixture was deacetylated by refluxing for 2 hours to give N,N'-bis(4-tolyl)-1,3-phenylenediamine represented by the formula (d).

Then, 7.2 g of N,N'-bis(4-tolyl)-1,3-phenylenediamine (d), 13.0 g of 4-n-butyliodobenzene of the formula (e), 13.8 g of potassium carbonate and 1 g of copper powder were added in 150 ml of nitrobenzene, and the mixture was refluxed while a nitrogen gas was blowing into this reaction system under vigorous stirring for 24 hours. The water produced in the reaction was removed out of the reaction system by azeotropic distillation with nitrobenzene, similar to the above case.

After the reaction solution was cooled, the inorganic substance was filtered off. Further, nitrobenzene was distilled off by steam distillation to give the residue, which was dissolved in cyclohexane. The resulting solution was purified by subjecting to silica gel column chromatography and cyclohexane was distilled off to give a white precipitate. Then, the white precipitate was recrystallized from n-hexane to give N,N'-bis(4-tolyl)-N,N'-bis(4-n-butylphenyl)-1,3-phenylenediamine of the formula (Ia) as an objective product (7.3 g, yield: 27.3 %, melting point: 93.3 °C).

The results of the infrared spectroscopic analysis of N,N'-bis(4-tolyl)-N,N'-bis(4-n-butylphenyl)-1,3-phenylenediamine are shown in Fig. 1. Further, the results of the elemental analysis are shown below.

| Elemental analysis (%), | | | |
|---|---|---|---|
| Calcd.: | C, 86.89; | H, 8.04; | N, 5.07 |
| Found: | C, 86.84; | H, 8.01; | N, 5.08 |

Synthesis Example 2

According to the same manner as that described in Synthesis Example 1, 7.2 g of N,N'-bis(4-tolyl)-1,3-phenylenediamine represented by the formula (d), obtained according to the same manner as that described in Synthesis Example 1, was reacted with 12.3 g of 4-isopropyliodobenzene represented by the formula (g):

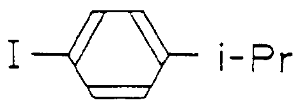

(g)

and the reaction product was separated, purified and recrystallized to give N,N'-bis(4-tolyl)-N,N'-bis(4-isopropylphenyl)-1,3-phenylenediamine represented by the formula (1b) (7.1 g, yield: 29.1 %, melting point: 120 °C).

The results of the infrared spectroscopic analysis of N,N'-bis(4-tolyl)-N,N'-bis(4-isopropylphenyl)-1,3-phenylenediamine are shown in Fig. **2**. Further, the results of the elemental analysis are shown below.

| Elemental analysis (%), | | | |
|---|---|---|---|
| Calcd.: | C, 86.96; | H, 7.70; | N, 5.34 |
| Found: | C, 86.95; | H, 7.67; | N, 5.33 |

Synthesis Example 3

Phenylenediamine compound represented by the formula (1c) was obtained from the corresponding starting compound in the same manner as in Synthesis Example 1. mp: 139.4°C

| Elemental analysis (%) | | | |
|---|---|---|---|
| Calcd.: | C, 88.53; | H, 6.55; | N, 4.92 |
| Found: | C, 88.61; | H, 6.51; | N, 4.96 |

Electrophotosensitive material

Examples 1 to 5 and Comparative Examples 1 to 4

(Single-layer type photosensitive material for digital light source)

5 parts by weight of a phthalocyanine pigment as the electric charge generating material, 50 parts by weight of a compound which belongs to the m-phenylenediamine derivative represented by the general formula (1) as the hole transferring material, 30 parts by weight of 3,5-dimethyl-3',5'-di-tert-butyldiphenoquinone represented by the formula (Q):

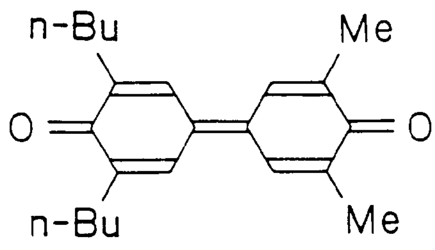

(Q)

as the electron transferring material and 100 parts by weight of polycarbonate as the binding resin were mixed and dispersed with 800 parts by weight of tetrahydrofuran as the solvent using a ball mill for 50 hours to prepare

a coating solution for single-layer type photosensitive layer. Then, this coating solution was applied on an aluminum tube as the conductive substrate by a dip coating method, followed by hot-air drying at 100 °C for 60 minutes to give a single-layer type photosensitive material for digital light source which has a single-layer type photosensitive layer of 15 to 20 μm in thickness.

Examples 6 and 7

(Multi-layer type photosensitive material for digital light source)

2 parts by weight of a phthalocyanine pigment as the electric charge generating material and 1 part by weight of polyvinyl butyral as the binding resin were mixed and dispersed with 120 parts by weight of dichloromethane as the solvent using a ball mill to prepare a coating solution for electric charge generating layer. Then, this coating solution was applied on an aluminum tube as the conductive substrate by a dip coating method, followed by hot-air drying at 100 °C for 60 minutes to form an electric charge generating layer of 0.5 μm in thickness.

Then, 80 parts by weight of a compound according to the m-phenylenediamine derivative represented by the formula (1) as the hole transferring material and 100 parts by weight of polycarbonate as the binding resin were mixed and dispersed with 800 parts by weight of benzene as the solvent using a ball mill to prepare a coating solution for electric charge transferring layer. Then, this coating solution was applied on the electric charge generating layer by a dip coating method, followed by hot-air drying at 90 °C for 60 minutes to form an electric charge transferring layer of 15 μm in thickness, thereby affording a multi-layer type photosensitive material for digital light source.

The respective compounds of the electric charge generating material and hole transferring material used in the above Examples and Comparative Examples are shown by the number of the compounds in Tables 1 and 2, respectively. Further, the m-phenylenediamine derivative (4d) used in Comparative Example 4 is the following compound.

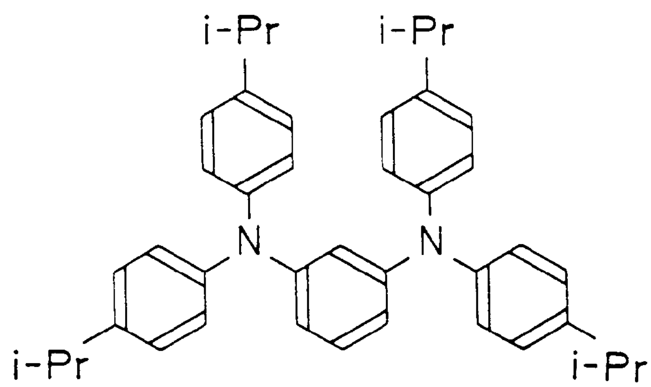

(4d)

The following test (I) of initial electric characteristics was conducted as to the photosensitive materials of the above Examples and Comparative Examples, and their characteristics were evaluated.

Test (I) of initial electric characteristics

By using a drum sensitivity tester manufactured by GENTEC Co., a voltage was applied on the surface of the photosensitive materials of the respective Examples and Comparative Examples to charge the surface at +700 V in case of the single-layer type and at -700 V in case of the multilayer type, respectively. Then, monochromatic light having a wavelength of 780 nm (half-width: 20 nm) and a light intensity of 16 μW/cm $^2$ from white light of a halogen lamp as an exposure light source through a band-pass filter was irradiated on the surface of the photosensitive material for 80 msec. (irradiation time). Further, a surface potential at the time at which 330 msec. has passed since the beginning of exposure was measured as a potential after exposure $V_L$ (V). Since the photosensitive material of Comparative Example 2 using the compound of the formula (4a) was crystallized when the photosensitive layer is formed, the test was not conducted.

The results of the single-layer type photosensitive material and those of the multi-layer type photosensitive material are shown in Table 1 and Table 2, respectively.

Table 1

| | Electric charge generating material | Hole transferrinlg material | Electron transferring material | $V_L$(V) |
|---|---|---|---|---|
| Example 1 | G1 | 1a | Q | +218 |
| Example 2 | G1 | 1b | Q | +215 |
| Example 3 | G1 | 1c | Q | +220 |
| Example 4 | G2 | 1a | Q | +223 |
| Example 5 | G2 | 1b | Q | +220 |
| Com.Example 1 | G1 | 4b | Q | +253 |
| Com.Example 2 | G1 | 4a | Q | * |
| Com.Example 3 | G1 | 4c | Q | +271 |
| Com.Example 4 | G1 | 4d | Q | +279 |

*: not measured because of crystallization

Table 2

| | Electric charge generating material | Hole transferring material | Electron transferring material | $V_L$(V) |
|---|---|---|---|---|
| Example 6 | G1 | 1a | Q | -148 |
| Example 7 | G1 | 1b | Q | -143 |

As is apparent from the results in Table 1, the photosensitive materials of Examples 1 to 5 are superior in sensitivity characteristics to the conventional photosensitive materials of Comparative Examples 1 and 3 using the compounds represented by the formulas (4b) and (4c) and the photosensitive material of Comparative Example 4 using the compound represented by the formula (4d) wherein the substituents $R^1$ to $R^4$ are the same isopropyl groups in the general formula (1), because of their low potential after exposure $V_L$ (V).

As is apparent from the results in Table 2, the photosensitive materials of Examples 6 and 7 are superior in sensitivity characteristics because of their low potential after exposure $V_L$ (V).

Examples 8 to 19 and Comparative Examples 5 to 16

(Single-layer type photosensitive material for analog light source)

5 parts by weight of an azo pigment as the electric charge generating material, 50 parts by weight of a compound which belongs to a m-phenylenediamine derivative represented by the general formula (1) as the hole transferring material, 30 parts by weight of 3,5-dimethyl-3',5'-di-tert-butyldiphenoquinone of the formula (Q) as the electron transferring material and 100 parts by weight of polycarbonate as the binding resin were mixed and dispersed with 800 parts by weight of tetrahydrofuran as the solvent using a ball mill for 50 hours to prepare a coating solution for single-layer type photosensitive layer. Then, this coating solution was applied on an aluminum tube as the conductive substrate by a dip coating method, followed by hot-air drying at 100 °C for 60 minutes to give a single-layer type photosensitive material for digital light source, which has a single-layer type photosensitive layer of 15 to 20 μm in thickness.

The following test (II) of initial electric characteristics was conducted as to the photosensitive materials of the above Examples and Comparative Examples, and their characteristics were evaluated.

Test (II) of initial electric characteristics

By using a drum sensitivity tester manufactured by GENTEC Co., a voltage was applied on the surface of the electrophotosensitive materials of the respective Examples and Comparative Examples to charge the surface at +700 V. Then, white light (light intensity: 147 $\mu W/cm^2$) of a halogen lamp as an exposure light source was irradiated on the surface of the photosensitive material for 50 msec. (irradiation time) and the time which is necessary for the above surface potential to be reduced to half, i.e. +350 V was measured, thereby calculating a half-life exposure $E_{1/2}$ ($\mu J/cm^2$). Further, a surface potential at the time at which 330 msec. has passed since the beginning of exposure was measured as a potential after exposure $V_L$ (V). Since the photosensitive materials of Comparative Examples 5, 9, 12 and 15 using the compound of the formula (4a) were crystallized when the photosensitive layer is formed, the test was not conducted.

The results are shown in Tables 3 and 4.

Table 3

| | Electric charge generating material | Hole transferring material | Electron transferring material | $V_L$(V) |
|---|---|---|---|---|
| Example 8 | G4 | 1a | Q | +205 |
| Example 9 | G4 | 1b | Q | +210 |
| Example 10 | G4 | 1c | Q | +216 |
| Example 11 | G5 | 1a | Q | +203 |
| Example 12 | G5 | 1b | Q | +206 |
| Example 13 | G5 | 1c | Q | +211 |
| Example 14 | G6 | 1a | Q | +213 |
| Example 15 | G6 | 1b | Q | +211 |
| Example 16 | G6 | 1c | Q | +220 |
| Example 17 | G7 | 1a | Q | +221 |
| Example 18 | G7 | 1b | Q | +224 |
| Example 19 | G7 | 1c | Q | +222 |

Table 4

| | Electric charge generating material | Hole transferring material | Electron transferring material | $V_L(V)$ |
|---|---|---|---|---|
| Com.Example 5 | G4 | 4b | Q | +249 |
| Com.Example 6 | G4 | 4a | Q | * |
| Com.Example 7 | G4 | 4c | Q | +257 |
| Com.Example 8 | G5 | 4b | Q | +254 |
| Com.Example 9 | G5 | 4a | Q | * |
| Com.Example 10 | G5 | 4c | Q | +244 |
| Com.Example 11 | G6 | 4b | Q | +249 |
| Com.Example 12 | G6 | 4a | Q | * |
| Com.Example 13 | G6 | 4c | Q | +259 |
| Com.Example 14 | G7 | 4b | Q | +260 |
| Com.Example 15 | G7 | 4a | Q | * |
| Com.Example 16 | G7 | 4c | Q | +254 |

*: not measured because of crystallization

As is apparent from the results in Tables 3 and 4, the photosensitive materials of Example 8 to 19 are superior in sensitivity characteristics to the conventional photosensitive materials of the respective Comparative Examples using the compounds represented by the formulas (4b) and (4c), because of their low potential after exposure $V_L$ (V).

Synthesis Example 4

According to the same manner as in Synthesis Example 1, 7.2 g of N,N'-bis(4-tolyl)-1,3-phenylenediamine represented by the formula (d), obtained in the same manner as in Synthesis Example 1, was reacted with 14.01 g of 4-phenyliodobenzene represented by the formula (f), and the reaction product was separated, purified and recrystallized to give N,N'-bis(4-tolyl)-N,N'-bis(4-biphenylyl)-1,3-phenylenediamine represented by

the formula (2a) (3.59 g, yield: 24.2 %).
mp: 148.4 °C

The results of the infrared spectroscopic analysis of N,N'-bis(4-tolyl)-N,N'-bis(4-biphenylyl)-1,3-phenylenediamine are shown in Fig. **3**. Further, the results of the elemental analysis are shown below.

| Elemental analysis (%), | | | |
|---|---|---|---|
| Calcd.: | C, 89.14; | H, 6.13; | N, 4.73 |
| Found: | C, 89.01; | H, 6.14; | N, 4.85 |

Further, the hole transferring capability of the above compound was evaluated by the following TOF method.

TOF method

A sample compound and polycarbonate as the binding resin (weight ratio: 1:1) were dissolved in tetrahydrofuran as the solvent to prepare a coating solution, which was applied on the surface of an aluminum sheet, followed by drying to form a sample membrane (thickness: 10 μm) wherein the sample compound is dispersed in the binding resin. Thereafter, gold was vacuum-deposited thereon to form a translucent gold electrode layer.

Then, a $N_2$ laser pulse having a wavelength of 337 nm was irradiated on the sample membrane for 3 ns through the translucent gold electrode layer while applying an electric field (electric field strength: 30 V/μm) between the aluminum sheet and the translucent gold electrode layer, and a mobility of electric charge ($cm^2$/V second) was measured to evaluate the hole transferring capability.

The results are shown in Table 5, together with the measurement results of the compound represented by the formula (4b).

Table 5

| Sample compound | Mobility of electric charge ($cm^2$/V second) |
|---|---|
| (2a) | $5.21 \times 10^{-5}$ |
| (4b) | $1.95 \times 10^{-5}$ |

As is apparent from the results in Table 5, the compound of the formula (2a), which belongs to the m-phenylenediamine derivative represented by the formula (2), is superior in hole transferring capability to the conventional compound represented by the formula (4a).

Synthesis Examples 5 to 12

Phenylenediamine compound represented by the formulas (2b) to (2i) were obtained from the corresponding starting compound in the same manner as in Synthesis Example 4.

Compound (2b)
mp: 146.3°C

| Elemental analysis (%) | | | |
|---|---|---|---|
| Calcd.: | C, 88.84; | H, 6.84; | N, 4.32 |
| Found: | C, 88.53; | H, 6.86; | N, 4.33 |

Compound (2c)
mp: 147.1°C

Elemental analysis (%)

Calcd.: C, 84.57; H, 5.81; N, 4.49

Found: C, 84.61; H, 5.79; N, 4.51

Compound (2d)
mp: 159.2°C

| Elemental analysis (%) | | | |
|---|---|---|---|
| Calcd.: | C, 79.60; | H, 4.78; | N, 4.42 |
| Found: | C, 79.62; | H, 4.81; | N, 4.39 |

Compound (2e)
mp: 176.5°C

| Elemental analysis (%) | | | |
|---|---|---|---|
| Calcd.: | C, 89.06; | H, 6.32; | N, 4.62 |
| Found: | C, 89.12; | H, 6.28; | N, 4.61 |

Compound (2f)
mp: 120.5°C

| Elemental analysis (%) | | | |
|---|---|---|---|
| Calcd.: | C, 89.06; | H, 6.32; | N, 4.62 |
| Found: | C, 89.01; | H, 6.35; | N, 4.59 |

Compound (2g)
mp: 140.5°C

| Elemental analysis (%) | | | |
|---|---|---|---|
| Calcd.: | C, 84.24; | H, 5.63; | N, 4.47 |
| Found: | C, 84.41; | H, 5.59; | N, 4.49 |

Compound (2h)
mp: 192.0°C

Elemental analysis (%)

Calcd.: C, 87.47; H, 5.71; N, 6.81

Found: C, 87.51; H, 5.76; N, 6.90

Compound (2i)
mp: 116.3°C

| Elemental analysis (%) | | | |
|---|---|---|---|
| Calcd.: | C, 86.77; | H, 6.15; | N, 4.50 |
| Found: | C, 85.99; | H, 6.21; | N, 4.45 |

Electrophotosensitive material Examples 20 to 29 and Comparative Examples 17 and 18

(Single-layer type photosensitive material for digital light source)

5 parts by weight of a phthalocyanine pigment as the electric charge generating material, 100 parts by weight of a compound which belong to the m-phenylenediamine derivative represented by the general formula (2) as the hole transferring material and 100 parts by weight of polycarbonate as the binding resin were mixed and dispersed with 800 parts by weight of tetrahydrofuran using a ball mill for 50 hours to prepare a coating solution for single-layer type photosensitive layer. Then, this coating solution was applied on an aluminum tube as the conductive substrate by a dip coating method, followed by hot-air drying at 110 °C for 30 minutes to give a single-layer type photosensitive material for digital light source, which has a single-layer type photosensitive layer of 25 μm in thickness.

Examples 30 to 39 and Comparative Examples 19 and 20

(Multi-layer type photosensitive material for digital light source)

2.5 parts by weight of a phthalocyanine pigment as the electric charge generating material and 1 part by weight of polyvinyl butyral as the binding resin were mixed and dispersed with 15 parts by weight of tetrahydrofuran using a ball mill to prepare a coating solution for electric charge generating layer. Then, this coating solution was applied on an aluminum tube as the conductive substrate by a dip coating method, followed by hot-air drying at 110 °C for 30 minutes to form an electric charge generating layer of 0.5 μm in film thickness.

Then, 1 part by weight of a compound which belongs to the m-phenylenediamine derivative represented by the general formula (2) as the hole transferring material and 1 part by weight of polycarbonate as the binding resin were mixed and dispersed with 10 parts by weight of tetrahydrofuran using a ball mill to prepare a coating solution for electric charge transferring layer. Then, this coating solution was applied on the electric charge generating layer by a dip coating method, followed by hot-air drying at 110 °C for 30 minutes to form an electric charge transferring layer of 20 μm in thickness, thereby affording a multi-layer type photosensitive material for digital light source.

The respective compounds of electric charge generating material and hole transferring material used in the above Examples and Comparative Examples are shown by the number of the compounds in Tables 6 and 7.

The following tests were conducted as to the respective photosensitive materials of the above Examples and Comparative Examples, and their characteristics were evaluated.

Test (III) of initial electric characteristics

By using a drum sensitivity tester manufactured by GENTEC Co., a voltage was applied on the surface of the photosensitive materials of the respective Examples and Comparative Examples to charge the surface at +700 ±20 V in case of the single-layer type and at -700 ±20 V in case of the multilayer type, respectively. Then, a surface potential at the time of charging was measured as an initial surface potential $V_0$ (V).

Then, monochromatic light having a wavelength of 780 nm (half-width: 20 nm) and a light intensity of 10 $\mu J/cm^2$ from white light of a halogen lamp as an exposure light source through a band-pass filter was irradiated on the surface of the photosensitive material for 1.5 seconds (irradiation time), and a surface potential at the time at which 0.5 seconds has passed since the beginning of exposure was measured as a residual potential $V_r$ (V). Further, an exposure which is necessary for the surface potential to be reduced to half of the initial surface potential $V_0$ was measured as a half-life exposure $E_{1/2}$ ($\mu J/cm^2$).

Test (I) of electric characteristics after aging

Among the photosensitive materials of the respective Examples and Comparative Examples, the single-layer type photosensitive material was fit in an electrostatic facsimile apparatus (Model TC-650, manufactured by Mita Kogyo Co., Ltd.) and the multi-layer type photosensitive material was fit in an electrostatic laser printer (Model LP-2080, manufactured by Mita Kogyo Co., Ltd.) and, after the image was formed 10,000 times, the electric characteristics were measured according to the same manner as that described above to determine a change $\Delta V_0$ (V) and $\Delta V_0$ (V) in initial surface potential $V_0$ and residual potential $V_r$ from the above initial value, respectively.

The results of the single-layer type photosensitive material and those of the multi-layer type photosensitive

material are shown in Table 6 and Table 7, respectively.

Table 6

| | Electric charge generating material | Hole transferring material | $V_0$(V) | $V_r$(V) | $E_{1/2}$ | $\Delta V_0$ (V) | $\Delta V_r$ (V) |
|---|---|---|---|---|---|---|---|
| Example 20 | G1 | 2a | 701 | 25 | 0.68 | -20 | +5 |
| Example 21 | G1 | 2b | 703 | 24 | 0.65 | -30 | ±0 |
| Example 22 | G1 | 2c | 709 | 29 | 0.74 | -25 | +10 |
| Example 23 | G1 | 2d | 713 | 32 | 0.76 | -20 | +5 |
| Example 24 | G1 | 2e | 706 | 27 | 0.64 | -20 | ±0 |
| Example 25 | G1 | 2f | 698 | 19 | 0.67 | -30 | +5 |
| Example 26 | G1 | 2g | 706 | 28 | 0.76 | -15 | ±0 |
| Example 27 | G1 | 2h | 711 | 29 | 0.70 | -20 | +10 |
| Example 28 | G1 | 2i | 701 | 25 | 0.72 | -10 | +5 |
| Example 29 | G2 | 2a | 704 | 25 | 0.64 | -15 | +10 |
| Com.Example 17 | G1 | 4b | 702 | 45 | 1.03 | -85 | +45 |
| Com.Example 18 | G2 | 4b | 708 | 43 | 0.95 | -80 | +35 |

As is apparent from the results in Table 6, the photosensitive materials of Example 20 to 29 are superior in sensitivity characteristics to the conventional photosensitive materials of Comparative Examples 17 and 18

using the compound represented by the formula (4b), because of their low residual potential $V_r$ (V) and small half-life exposure $E_{1/2}$ (μJ/cm²). Further, the photosensitive materials of the above respective Examples are superior in stability to the photosensitive materials of Comparative Examples 17 and 18, because of their small change $\Delta V_0$ (V) and $\Delta V_0$ (V) in initial surface potential $V_0$ and residual potential $V_r$ after aging.

Table 7

| | Electro charge generating material | Hole transferring material | $V_0$(V) | $V_r$(V) | $E_{1/2}$ | $\Delta V_0$ (V) | $\Delta V_r$ (V) |
|---|---|---|---|---|---|---|---|
| Example 30 | G1 | 2a | -707 | -10 | 0.48 | -5 | ±0 |
| Example 31 | G1 | 2b | -706 | -11 | 0.45 | -10 | ±0 |
| Example 32 | G1 | 2c | -712 | -14 | 0.54 | -5 | +5 |
| Example 33 | G1 | 2d | -698 | -16 | 0.56 | -20 | ±0 |
| Example 34 | G1 | 2e | -705 | -12 | 0.44 | -15 | -5 |
| Example 35 | G1 | 2f | -711 | -13 | 0.47 | -15 | ±0 |
| Example 36 | G1 | 2g | -701 | -15 | 0.56 | -5 | ±0 |
| Example 37 | G1 | 2h | -704 | -13 | 0.50 | -15 | +10 |
| Example 38 | G1 | 2i | -706 | -10 | 0.52 | -5 | +5 |
| Example 39 | G2 | 2a | -701 | -10 | 0.44 | -10 | +5 |
| Com.Example 19 | G1 | 4b | -703 | -20 | 0.83 | -55 | +35 |
| Com.Example 20 | G2 | 4b | -711 | -18 | 0.75 | -50 | +40 |

As is apparent from the results in Table 7, the photosensitive materials of Example 30 to 39 are superior in sensitivity characteristics to the conventional photosensitive materials of Comparative Examples 19 and 20 using the compound represented by the formula (4b), because of their low residual potential $V_r$ (V) and small half-life exposure $E_{1/2}$ ($\mu J/cm^2$). Further, the photosensitive materials of the above respective Examples are superior in stability to the photosensitive materials of Comparative Examples 19 and 20, because of their small change $\Delta V_0$ (V) and $\Delta V_r$ (V) in initial surface potential $V_0$ and residual potential $V_r$ after aging, respectively.

Examples 40 to 54 and Comparative Examples 21 to 25

(Single-layer type photosensitive material for analog light source)

5 parts by weight of a perylene or azo pigment as the electric charge generating material, 100 parts by weight of a compound which belong to the m-phenylenediamine derivative represented by the general formula (2) as the hole transferring material and 100 parts by weight of polycarbonate as the binding resin were mixed and dispersed with 800 parts by weight of tetrahydrofuran as the solvent using a ball mill for 50 hours to prepare a coating solution for single-layer type photosensitive layer. Then, this coating solution was applied on an aluminum tube as the conductive substrate by a dip coating method, followed by hot-air drying at 110 °C for 30 minutes to give a single-layer type photosensitive material for digital light source which has a single-layer type electrophotosensitive layer of 25 μm in thickness.

Examples 55 to 69 and Comparative Examples 26 to 30

(Multi-layer type photosensitive material for analog light source)

2.5 parts by weight of a perylene or azo pigment as the electric charge generating material and 1 part by weight of polyvinyl butyral as the binding resin were mixed and dispersed with 15 parts by weight of tetrahydrofuran as the solvent using a ball mill to prepare a coating solution for electric charge generating layer. Then, this coating solution was applied on an aluminum tube as the conductive substrate by a dip coating method, followed by hot-air drying at 110 °C for 30 minutes to form an electric charge generating layer of 0.5 μm in thickness.

Then, 1 part by weight of a compound which belongs to the m-phenylenediamine derivative represented by the general formula (2) as the hole transferring material and 1 part by weight of polycarbonate as the binding resin were mixed and dispersed with 10 parts by weight of tetrahydrofuran using a ball mill to prepare a coating solution for electric charge transferring layer. Then, this coating solution was applied on the electric charge generating layer by a dip coating method, followed by hot-air drying at 110 °C for 30 minutes to form an electric charge transferring layer of 20 μm in thickness, thereby affording a multi-layer type photosensitive material for analog light source.

The respective compounds of the electric charge generating material and hole transferring material used in the above Examples and Comparative Examples are shown by the number of the compounds in Tables 8 and 9.

The following tests were conducted as to the respective photosensitive materials of the above Examples and Comparative Examples, and their characteristics were evaluated.

Test (IV) of initial electric characteristics

By using a drum sensitivity tester manufactured by GENTEC Co., a voltage was applied on the surface of the photosensitive materials of the respective Examples and Comparative Examples to charge the surface at +700 ±20 V in case of the single-layer type and at -700 ±20 V in case of the multi-layer type, respectively. Then, a surface potential at the time of charging was measured as an initial surface potential $V_0$ (V).

Then, white light (light intensity: 10 lux) of a halogen lamp as an exposure light source was irradiated on the surface of the photosensitive material for 1.5 seconds (irradiation time), and a surface potential at the time at which 0.5 seconds has passed since the beginning of exposure was measured as a residual potential $V_r$ (V). Further, an exposure which is necessary for the surface potential to be reduced to half of the initial surface potential $V_0$ was measured as a half-life exposure $E_{1/2}$ (lux second).

Test (II) of electric characteristics after aging

Among the photosensitive materials of the respective Examples and Comparative Examples, the single-layer type photosensitive material was fit in an electrostatic copying machine (Model DC-2556, manufactured by Mita Kogyo Co., Ltd.) and the multi-layer type photosensitive material was fit in an electrostatic copying

machine modified with a negative changing specification (Model DC-2556, manufactured by Mita Kogyo Co., Ltd.) and, after the image was formed 10,000 times, the electric characteristics were measured according to the same manner as that described above to determine a change $\Delta V_0$ (V) and $\Delta V_r$ (V) in initial surface potential $V_0$ and residual potential $V_r$ from the above initial value, respectively.

The results of the single-layer type photosensitive material and those of the multi-layer type photosensitive material are shown in Table 8 and Table 9, respectively.

Table 8

| | Electro charge generating material | Hole transferring material | $V_0(V)$ | $V_r(V)$ | $E_{1/2}$ | $\Delta V_0$ (V) | $\Delta V_r$ (V) |
|---|---|---|---|---|---|---|---|
| Example 40 | G3 | 2a | 698 | 40 | 1.96 | -10 | +15 |
| Example 41 | G8 | 2a | 707 | 95 | 1.25 | -105 | +5 |
| Example 42 | G8 | 2e | 705 | 97 | 1.23 | -90 | +5 |
| Example 43 | G8 | 2g | 696 | 103 | 1.30 | -120 | +10 |
| Example 44 | G6 | 2a | 713 | 95 | 1.10 | -55 | +5 |
| Example 45 | G6 | 2b | 704 | 96 | 1.09 | -60 | +5 |
| Example 46 | G6 | 2e | 706 | 98 | 1.21 | -40 | ±0 |
| Example 47 | G6 | 2h | 703 | 95 | 1.18 | -85 | -5 |
| Example 48 | G9 | 2a | 699 | 90 | 0.98 | -40 | ±0 |
| Example 49 | G9 | 2c | 703 | 92 | 0.99 | -55 | ±0 |
| Example 50 | G9 | 2f | 696 | 95 | 1.02 | -60 | -5 |
| Example 51 | G9 | 2g | 714 | 98 | 1.11 | -50 | +5 |
| Example 52 | G9 | 2h | 707 | 96 | 1.08 | -55 | +5 |
| Example 53 | G9 | 2i | 701 | 95 | 1.06 | -60 | ±0 |
| Example 54 | G10 | 2a | 706 | 123 | 1.41 | -150 | +10 |
| Com.Example 21 | G3 | 4b | 707 | 186 | 2.45 | -95 | +60 |
| Com.Example 22 | G8 | 4b | 704 | 121 | 1.60 | -215 | +45 |
| Com.Example 23 | G6 | 4b | 707 | 124 | 1.68 | -180 | +50 |
| Com.Example 24 | G9 | 4b | 696 | 110 | 1.47 | -160 | +40 |
| Com.Example 25 | G10 | 4b | 701 | 145 | 2.06 | -250 | +55 |

As is apparent from the results in Table 8, the photosensitive materials of Example 40 to 54 are superior in sensitivity characteristics to the conventional photosensitive materials of Comparative Examples 21 to 25 using the compound represented by the formula (4b), because of their low residual potential $V_r$ (V) and small half-life exposure $E_{1/2}$ (lux second). Further, the photosensitive materials of the above respective Examples

are superior in stability to the photosensitive materials of Comparative Examples 21 to 25, because of their small change $\Delta V_0$ (V) and $\Delta V_r$ (V) in initial surface potential $V_0$ and residual potential $V_r$ after aging.

Table 9

| | Electro charge generating material | Hole transferring material | $V_0$(V) | $V_r$(V) | $E_{1/2}$ | $\Delta V_0$ (V) | $\Delta V_r$ (V) |
|---|---|---|---|---|---|---|---|
| Example 55 | G3 | 2a | -711 | -101 | 1.88 | -25 | +20 |
| Example 56 | G8 | 2a | -709 | -56 | 1.24 | -65 | +15 |
| Example 57 | G8 | 2e | -699 | -60 | 1.27 | -75 | +20 |
| Example 58 | G8 | 2g | -703 | -58 | 1.31 | -70 | +20 |
| Example 59 | G6 | 2a | -699 | -45 | 1.05 | -40 | ±0 |
| Example 60 | G6 | 2b | -697 | -50 | 1.18 | -25 | +5 |
| Example 61 | G6 | 2e | -705 | -42 | 1.01 | -50 | +5 |
| Example 62 | G6 | 2h | -710 | -46 | 1.10 | -55 | ±0 |
| Example 63 | G9 | 2a | -706 | -35 | 0.90 | -15 | ±0 |
| Example 64 | G9 | 2c | -699 | -40 | 0.95 | -20 | -5 |
| Example 65 | G9 | 2f | -698 | -37 | 0.93 | -20 | ±0 |
| Example 66 | G9 | 2g | -710 | -42 | 0.98 | -15 | ±0 |
| Example 67 | G9 | 2h | -711 | -40 | 0.94 | -10 | +5 |
| Example 68 | G9 | 2i | -705 | -38 | 0.95 | -25 | ±0 |
| Example 69 | G10 | 2a | -700 | -85 | 1.62 | -55 | +20 |
| Com.Example 26 | G3 | 4b | -711 | -156 | 2.56 | -40 | +65 |
| Com.Example 27 | G8 | 4b | -706 | -95 | 1.52 | -140 | +45 |
| Com.Example 28 | G6 | 4b | -696 | -105 | 1.73 | -120 | +40 |
| Com.Example 29 | G9 | 4b | -712 | -80 | 1.45 | -110 | +35 |
| Com.Example 30 | G10 | 4b | -705 | -137 | 1.98 | -90 | +50 |

As is apparent from the results in Table 9, the photosensitive materials of Example 55 to 69 are superior

in sensitivity characteristics to the conventional photosensitive materials of Comparative Examples 26 to 30 using the compound represented by the formula (4b), because of their low residual potential $V_r$ (V) and small half-life exposure $E_{1/2}$ (lux second). Further, the photosensitive materials of the above respective Examples are superior in stability to the photosensitive materials of Comparative Examples 26 to 30, because of their small change $\Delta V_0$(V) and $\Delta V_0$(V) in initial surface potential $V_0$ and residual potential $V_r$ after aging.

Electrophotosensitive material

Reference Example 1

(Synthesis of m-phenylenediamine derivative (3))

In the same manner as in Synthesis Example 1, 7.2 g of N,N'-bis(4-tolyl)-1,3-phenylenediamine represented by the formula (d), obtained in the same manner as in Synthesis Example 1, was reacted with 15.41 g of 4-(4-ethylphenyl)iodobenzenerepresented by the formula (h):

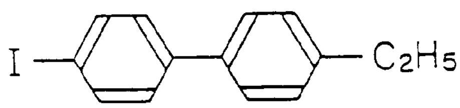

(h)

and the reaction product was separated, purified and recrystallized to give N,N'-bis(4-tolyl)-N,N'-bis[4-(4'-ethyl)biphenylyl)-1,3-phenylenediamine represented by the formula (3d) (3.75 g, yield: 23.1%).

The results of the infrared spectroscopic analysis of N,N'-bis(4-tolyl)-N,N'-bis[4-(4'-ethyl)biphenylyl)-1,3-phenylenediamine are shown in Fig. 4. Further, the results of the elemental analysis are shown below.

mp: 151.4°C

| Elemental analysis (%), | | | |
|---|---|---|---|
| Calcd.: | C, 88.84; | H, 6.84; | N, 4.32 |
| Found: | C, 88.63; | H, 6.94; | N, 4.33 |

Further, the hole transferring capability of the above compound was evaluated by the above TOF method.

The results are shown in Table 10, together with the measurement results of the compound represented by the formula (4b).

Table 10

| Sample compound | Mobility of electric charge ($cm^2$/V second) |
|---|---|
| (3d) | $3.39 \times 10^{-5}$ |
| (4b) | $1.95 \times 10^{-5}$ |

As is apparent from the results in Table 10, the compound of the formula (3d), which belongs to the m-phenylenediamine derivative represented by the formula (3), is superior in hole transferring capability to the conventional compound represented by the formula (4b).

Reference Examples 2 to 7

Phenylenediamine compound represented by the formulas (3a) to (3c) and (3e) to (3g) were obtained from the corresponding starting compound in the same manner as in Reference Example 1.

Compound (3a)

mp: 145.1°C

| Elemental analysis (%) | | | |
|---|---|---|---|
| Calcd.: | C, 88.30; | H, 6.46; | N, 5.28 |
| Found: | C, 88.27; | H, 6.51; | N, 5.26 |

Compound (3b)
mp: 156.5°C

Elemental analysis (%)

Calcd.: C, 88.19; H, 6.67; N, 5.15

Found: C, 88.23; H, 6.64; N, 5.12

Compound (3c)
mp: 148 .7°C

| Elemental analysis (%) | | | |
|---|---|---|---|
| Calcd.: | C, 89.15; | H, 6.13; | N, 4.73 |
| Found: | C, 89.06; | H, 6.14; | N, 4.71 |

Compound (3e)
mp: 133.6°C

| Elemental analysis (%) | | | |
|---|---|---|---|
| Calcd.: | C, 88.71; | H, 7.15; | N, 4.14 |
| Found: | C, 88.90; | H, 7.13; | N, 4.21 |

Compound (3f)
mp: 158.5°C

| Elemental analysis (%) | | | |
|---|---|---|---|
| Calcd.: | C, 88.77; | H, 6.99; | N, 4.23 |
| Found: | C, 88.66; | H, 6.96; | N, 4.26 |

Compound (3g)
mp: 201.2°C

| Elemental analysis (%) | | | |
|---|---|---|---|
| Calcd.: | C, 90.23; | H, 6.14; | N, 3.63 |
| Found: | C, 90.19; | H, 6.16; | N, 3.61 |

<u>Examples 70 to 77</u>

(Single-layer type photosensitive material for digital light source)

5 parts by weight of a phthalocyanine pigment as the electric charge generating material, 100 parts by weight of a compound which belong to the m-phenylenediamine derivative represented by the general formula (3) as the hole transferring material and 100 parts by weight of polycarbonate as the binding resin were mixed

and dispersed with 800 parts by weight of tetrahydrofuran using a ball mill for 50 hours to prepare a coating solution for single-layer type electrophotosensitive layer. Then, this coating solution was applied on an aluminum tube as the conductive substrate by a dip coating method, followed by hot-air drying at 110°C for 30 minutes to give a single-layer type photosensitive material for digital light source which has a single-layer type photosensitive layer of 25 μm in thickness.

The respective compounds of the electric charge generating material and hole transferring material used in the above Examples are shown by the number of the compounds in Table 11.

The above test (III) of initial electric characteristics and test (I) of electric characteristics after aging were conducted as to the respective photosensitive materials of the above Examples and Comparative Examples, and their characteristics were evaluated.

The results are shown in Table 11, together with those of the above Comparative Examples 17 and 18.

Table 11

| | Electro charge generating material | Hole transferring material | $V_0$(V) | $V_r$(V) | $E_{1/2}$ | $\Delta V_0$ (V) | $\Delta V_r$ (V) |
|---|---|---|---|---|---|---|---|
| Example 70 | G1 | 3a | 697 | 32 | 0.75 | -30 | +15 |
| Example 71 | G1 | 3b | 699 | 31 | 0.72 | -35 | +20 |
| Example 72 | G1 | 3c | 706 | 27 | 0.68 | -20 | +5 |
| Example 73 | G1 | 3d | 708 | 24 | 0.66 | -15 | +5 |
| Example 74 | G1 | 3e | 698 | 33 | 0.81 | -30 | +10 |
| Example 75 | G1 | 3f | 711 | 29 | 0.68 | -20 | ±0 |
| Example 76 | G1 | 3g | 708 | 27 | 0.74 | -10 | +5 |
| Example 77 | G2 | 3d | 699 | 20 | 0.62 | -20 | ±0 |
| Com.Example 17 | G1 | 4b | 702 | 45 | 1.03 | -85 | +45 |
| Com.Example 18 | G2 | 4b | 708 | 43 | 0.95 | -80 | +35 |

As is apparent from the results in Table 11, the photosensitive materials of Example 70 to 77 are superior in sensitivity characteristics to the conventional photosensitive materials of Comparative Examples 17 and 18 using the compound represented by the formula (4b), because of their low residual potential $V_r$ (V) and small half-life exposure $E_{1/2}$ (μJ/cm²). Further, the photosensitive materials of the above respective Examples are superior in stability to the photosensitive materials of Comparative Examples 17 and 18, because of their small

change $\Delta V_0(V)$ and $\Delta V_r(V)$ in initial surface potential $V_0$ and residual potential $V_r$ after aging.

Examples 78 to 85

(Single-layer type photosensitive material for analog light source)

5 Parts by weight of a perylene or azo pigment as the electric charge generating material, 100 parts by weight of a compound which belong to the m-phenylenediamine derivative represented by the general formula (3) as the hole transferring material and 100 parts by weight of polycarbonate as the binding resin were mixed and dispersed with 800 parts by weight of tetragydrofuran as the solvent using a ball mill for 50 hours to prepare a coating solution for single-layer type photosensitive layer. Then, this coating solution was applied on an aluminum tube as the conductive substrate by a dip coating method, followed by hot-air drying at 110°C for 30 minutes to give a single-layer type photosensitive material for digital light source, which has a single-layer type photosensitive layer of 25 μm in thickness.

The respective compounds of the electric charge generating material and hole transferring material used in the above Examples are shown by the number of the compounds in Table 12.

The above test (IV) of initial electric characteristics and test (II) of electric characteristics after aging were conducted as to the respective photosensitive materials of the above Examples and Comparative Examples, and their characteristics were evaluated.

The results are shown in Table 12, together with those of the above Comparative Examples 21 to 25.

Table 12

| | Electro charge generating material | Hole transferring material | $V_0$(V) | $V_r$(V) | $E_{1/2}$ | $\Delta V_0$ (V) | $\Delta V_r$ (V) |
|---|---|---|---|---|---|---|---|
| Example 78 | G3 | 3d | 712 | 138 | 1.94 | -20 | +10 |
| Example 79 | G8 | 3d | 698 | 94 | 1.18 | -85 | +10 |
| Example 80 | G8 | 3f | 701 | 105 | 1.22 | -80 | +5 |
| Example 81 | G6 | 3a | 708 | 106 | 1.23 | -110 | +10 |
| Example 82 | G6 | 3c | 707 | 96 | 1.15 | -60 | +5 |
| Example 83 | G6 | 3e | 705 | 102 | 1.26 | -55 | +5 |
| Example 84 | G6 | 3g | 712 | 104 | 1.25 | -70 | ±0 |
| Example 85 | G9 | 3d | 705 | 92 | 0.98 | -55 | +5 |
| Com.Example 21 | G3 | 4b | 707 | 186 | 2.45 | -95 | +60 |
| Com.Example 22 | G8 | 4b | 704 | 121 | 1.60 | -215 | +45 |
| Com.Example 23 | G6 | 4b | 707 | 124 | 1.68 | -180 | +50 |
| Com.Example 24 | G9 | 4b | 696 | 110 | 1.47 | -160 | +40 |
| Com.Example 25 | G10 | 4b | 701 | 145 | 2.06 | -250 | +55 |

As is apparent from the results in Table 12, the photosensitive materials of Example 78 to 85 are superior in sensitivity characteristics to the conventional photosensitive materials of Comparative Examples 21 to 25 using the compound represented by the formula (4b), because of their low residual potential $V_r$(V) and small half-life exposure $E_{1/2}$ (lux second). Further, the photosensitive materials of the above respective Examples

are superior in stability to the photosensitive materials of Comparative Examples 21 to 25, because of their small change $\Delta V_0(V)$ and $\Delta V_r(V)$ in initial surface potential $V_0$ and residual potential $V_r$ after aging.

## Claims

1. A m-phenylenediamine derivative represented by the general formula (1):

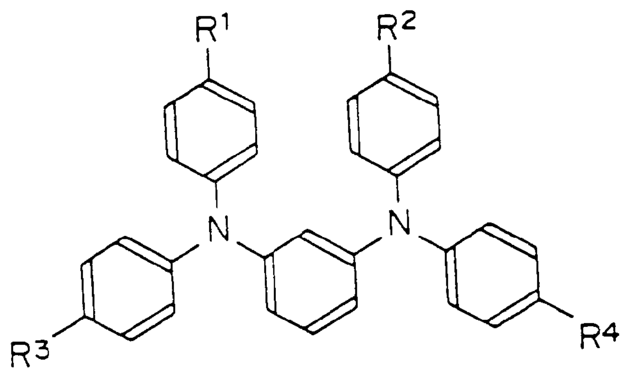

(1)

wherein $R^1$ and $R^2$ are the same or different and each indicates a hydrogen atom or an alkyl group which may have one or more substituents; and $R^3$ and $R^4$ are the same or different and each indicates a straight-chain or branched alkyl group having 3 to 5 carbon atoms; provided that $R^1$, $R^2$, $R^3$ and $R^4$ are not the same alkyl groups, simultaneously.

2. An electrophotosensitive material comprising a photosensitive layer containing a m-phenylenediamine derivative represented by the general formula (1) defined in claim 1, on a conductive substrate.

3. The electrophotosensitive material according to claim 2, wherein the photosensitive layer is a single-layer type photosensitive layer containing an electric charge generating material and a m-phenylenediamine derivative represented by the general formula (1) as an electric charge transferring material.

4. The electrophotosensitive material according to claim 2, wherein the photosensitive layer is a multi-layer type photosensitive layer comprising an electric charge generating layer containing an electric charge generating material and an electric charge transferring layer containing a m-phenylenediamine derivative represented by the general formula (1) as an electric charge transferring material, both layers being laminated mutually.

5. A m-phenylenediamine derivative represented by the general formula (2):

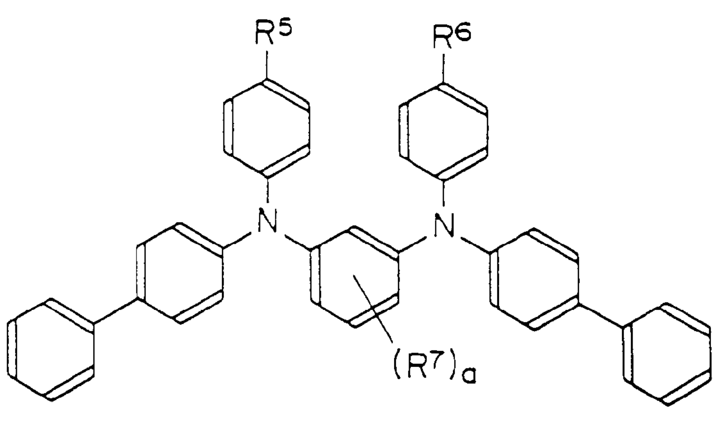

(2)

wherein $R^5$ and $R^6$ are the same or different and each indicates an alkyl group which may have one or more substituents, an alkoxy group which may have one or more substituents or a halogen atom; $R^7$ is

a hydrogen atom, an alkyl group which may have one or more substituents, an alkoxy group which may have one or more substituents, an aryl group which may have one or more substituents, a halogen atom, a cyano group or a nitro group; and
a is an integer from 1 to 4.

6. An electrophotosensitive material comprising a photosensitive layer containing a m-phenylenediamine derivative represented by the general formula (2) defined in claim 5, on a conductive substrate.

7. The electrophotosensitive material according to claim 6, wherein the photosensitive layer is a single-layer type photosensitive layer containing an electric charge generating material and a m-phenylenediamine derivative represented by the general formula (2) as an electric charge transferring material.

8. The electrophotosensitive material according to claim 6, wherein the photosensitive layer is a multi-layer type photosensitive layer comprising an electric charge generating layer containing an electric charge generating material and an electric charge transferring layer containing a m-phenylenediamine derivative represented by the general formula (2) as an electric charge transferring material, both layers being laminated mutually.

9. An electrophotosensitive material comprising a single-layer type photosensitive layer containing an electric charge generating material and a m-phenylenediamine derivative represented by the general formula (3):

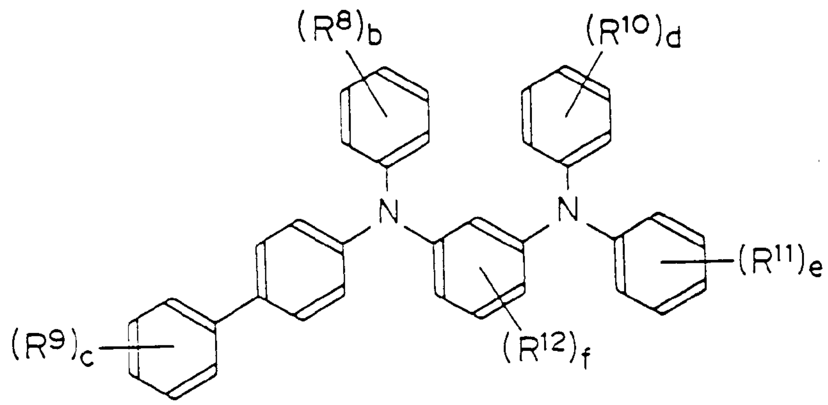

(3)

wherein $R^8$, $R^9$, $R^{10}$ and $R^{11}$ are the same or different and each indicates a hydrogen atom, an alkyl group which may have one or more substituents, an alkoxy group which may have one or more substituents, an aryl group which may have one or more substituents or a halogen atom; $R^{12}$ is a hydrogen atom, an alkyl group which may have one or more substituents, an alkoxy group which may have one or more substituents, an aryl group which may have one or more substituents, a halogen atom, a cyano group or a nitro group; b, c, d and e independently indicate an integer from 1 to 5; and f is an integer of 1 to 4, as an electric charge transferring material, on a conductive substrate.

FIG. 1

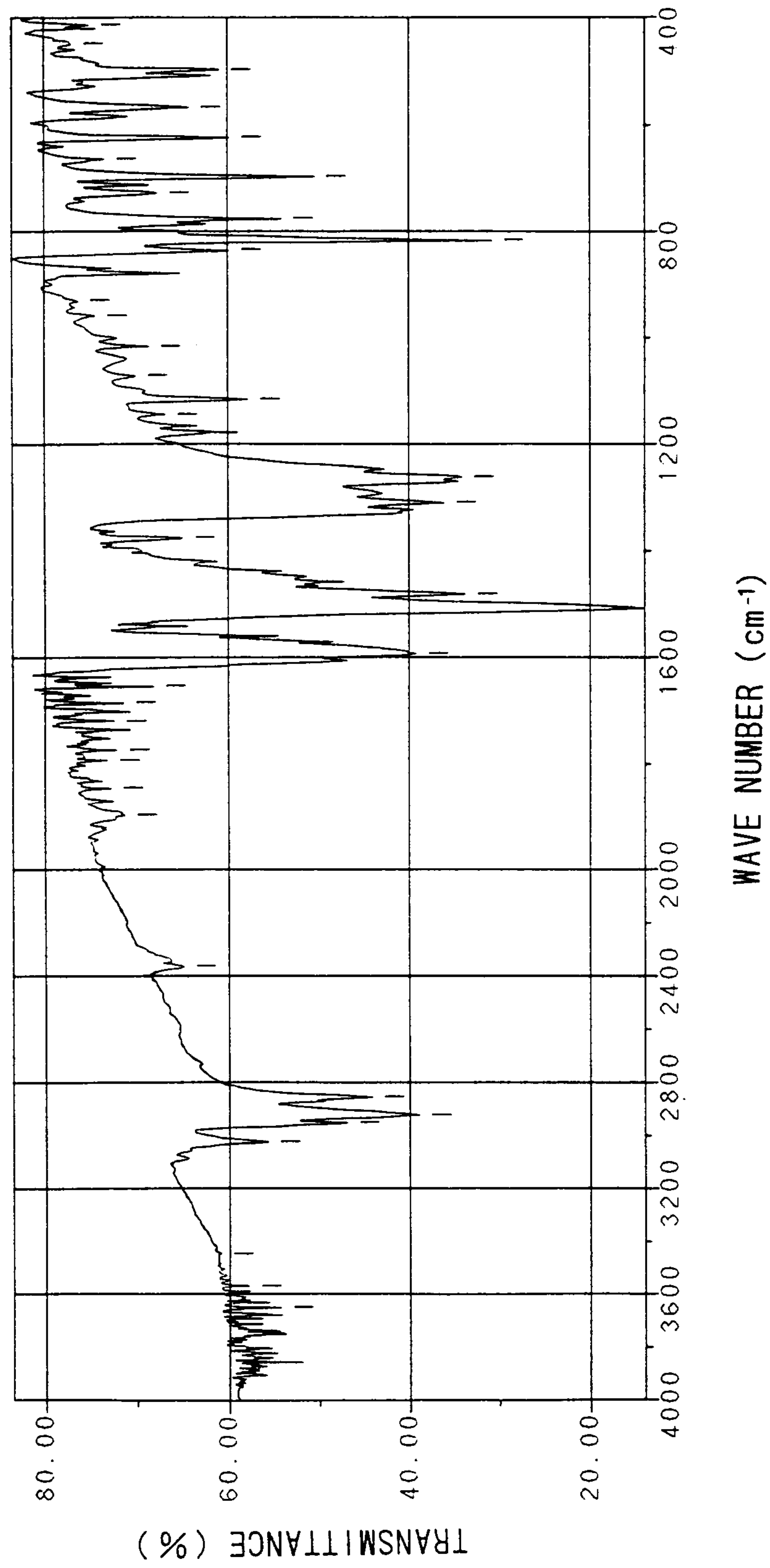

TRANSMITTANCE (%)
80.00
60.00
40.00
20.00
WAVE NUMBER (cm-1)
4000
3600
3200
2800
2400
2000
1600
1200
800
400

FIG. 2

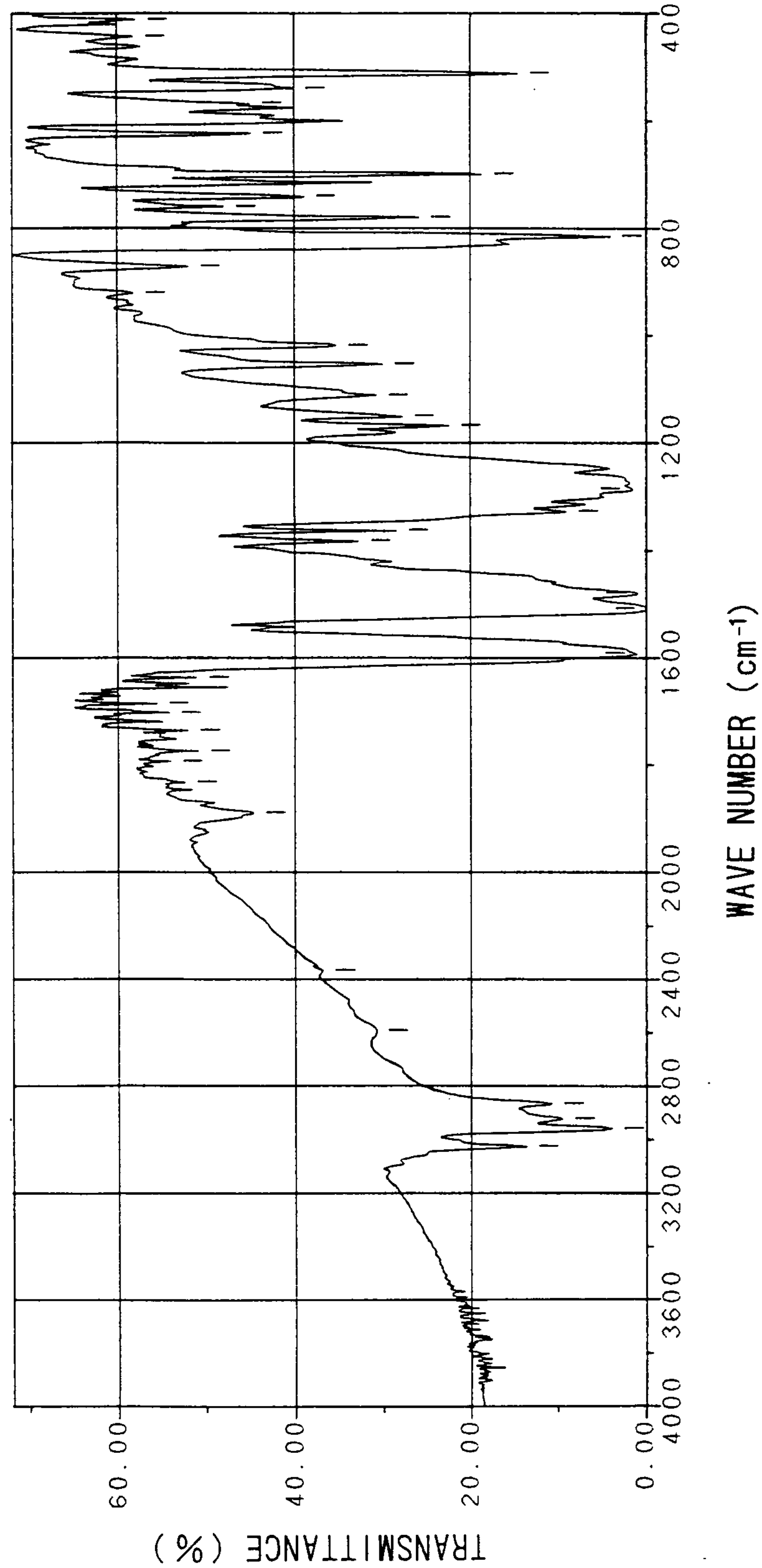
TRANSMITTANCE (%)
0.00
20.00
40.00
60.00
WAVE NUMBER (cm⁻¹)
4000
3600
3200
2800
2400
2000
1600
1200
800
400

F I G. 3

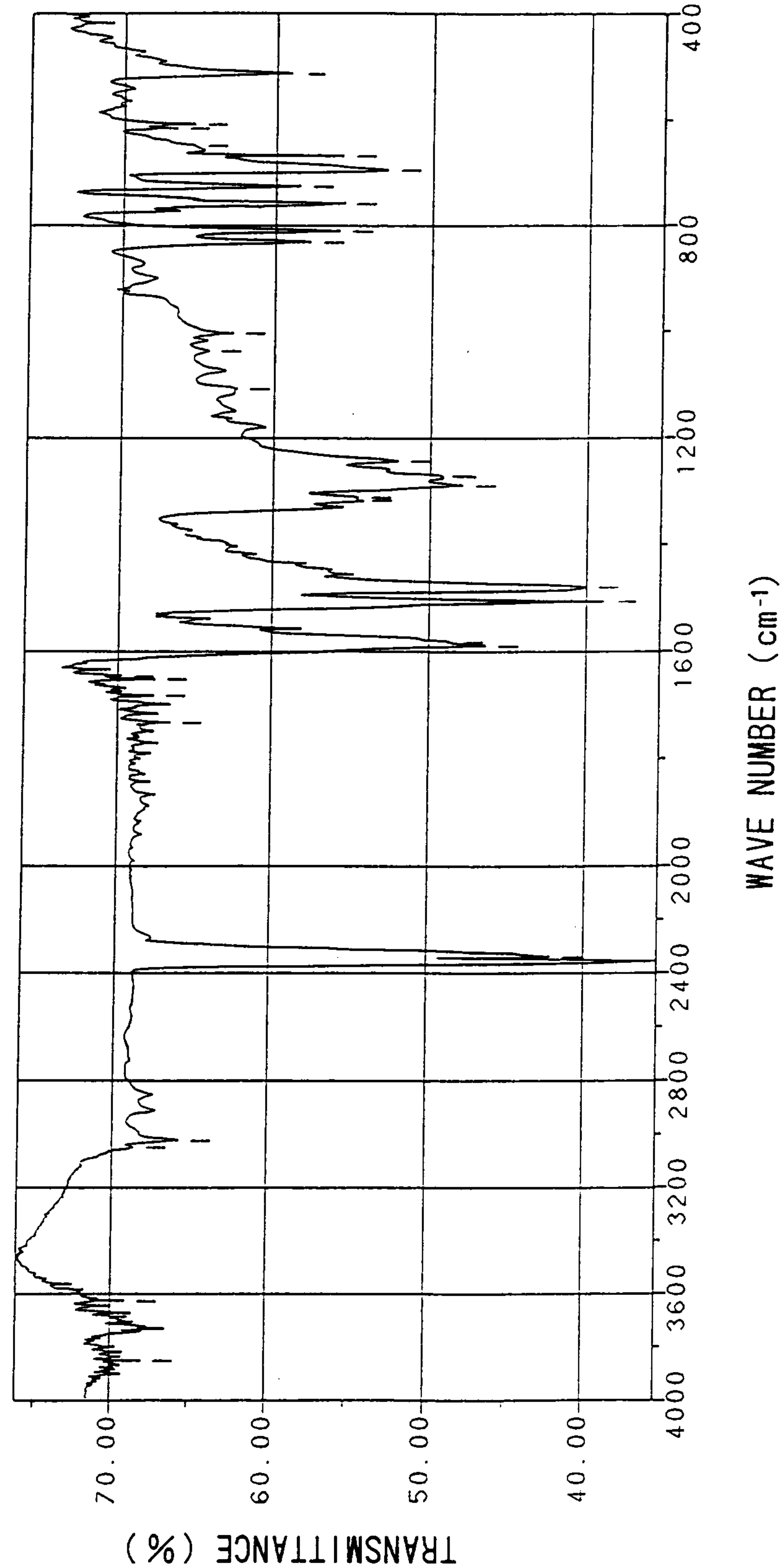
TRANSMITTANCE (%)
70.00
60.00
50.00
40.00
WAVE NUMBER (cm-1)
4000
3600
3200
2800
2400
2000
1600
1200
800
400

FIG. 4

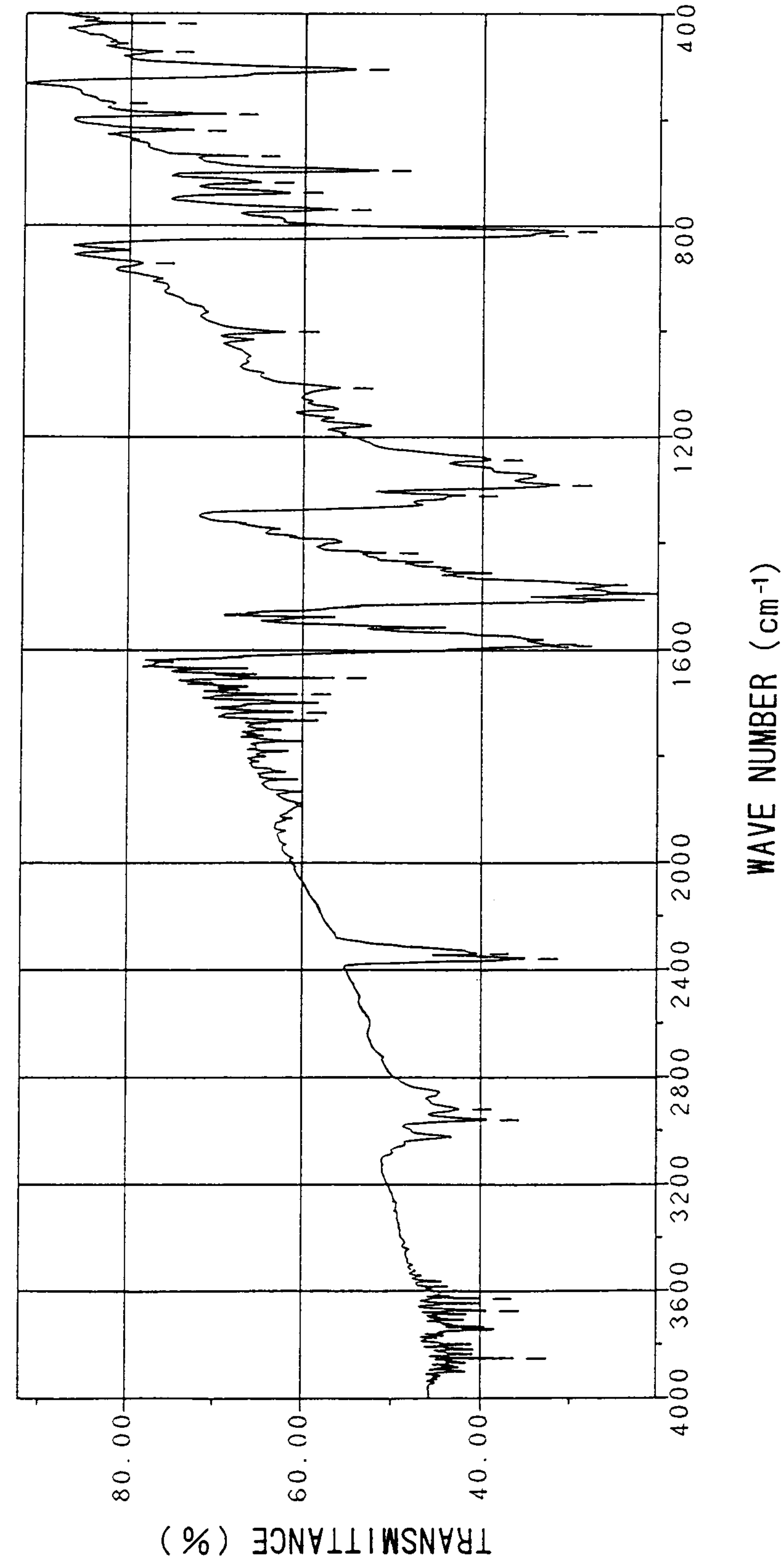
TRANSMITTANCE (%)
80.00
60.00
40.00
WAVE NUMBER (cm-1)
4000
3600
3200
2800
2400
2000
1600
1200
800
400